# FASCICULE DE BREVET EUROPEEN

(11) **EP 4 045 566 B1**
(45) Date de publication et mention de la délivrance du brevet: **06.12.2023**
(21) Numéro de dépôt: 20799786.7
(22) Date de dépôt: 15.10.2020
(51) Int. Cl.: C08G 69/44, C08L 77/12, C08L 91/00, A61K 8/92, A61K 8/88, A61K 47/14, A61K 47/34, A61K 47/42, A61Q 19/00, C08G 69/26, C08G 63/06, A61K 9/06, A61K 47/44

(54) **POLYAMIDES GÉLIFIANTS BIOSOURCÉS**
GELIERENDE POLYAMIDE AUF BIOBASIS
BIOSOURCED GELLING POLYAMIDES

(30) Priorité: 16.10.2019 FR 1911530
(43) Date de publication de la demande: 24.08.2022
(73) Titulaire: Polymerexpert SA, 33600 Pessac (FR)
(72) Inventeur: DOLATKHANI, Marc, 33610 CESTAS (FR); LUTZ, Eric, 33600 PESSAC (FR); HECHT, Marie Odile, 33600 PESSAC (FR); SISCAN, Olga, 33600 PESSAC (FR); SALVADO, Victor, 33700 MERIGNAC (FR); PAGNOUX, Anne, 33114 LE BARP (FR)
(74) Mandataire: Santarelli
(86) Numéro de dépôt international: PCT/FR2020/051849
(87) Numéro de publication internationale: WO 2021/074541

(56) Documents cités:
- US-A- 5 703 177
- US-A- 6 111 055
- DATABASE WPI Week 198730 Thomson Scientific, London, GB; AN 1987-209341 XP002799536, -& JP S62 135521 A (DAICEL CHEM IND LTD) 18 juin 1987 (1987-06-18)

## Description

L'invention concerne de nouveaux dérivés polyamides gélifiants d'origine biosourcée, leur procédé de préparation et l'élaboration de nouveaux gels en milieux organiques (huiles) à partir de ceux-ci. L'invention concerne également les gels formés à partir de ces polyamides gélifiants, ainsi que les compositions les contenant, en particulier les compositions cosmétiques et pharmaceutiques.

Le procédé selon l'invention permet d'obtenir un composé polyamide apte à former des gels ayant les propriétés recherchées, en mettant en oeuvre deux étapes de fabrication. Lors d'une première étape, un dérivé amide de masse molaire faible est d'abord obtenu par fonctionnalisation d'un diacide par une diamine, conférant les propriétés gélifiantes du composé de l'invention, le diacide et la diamine pouvant être biosourcés. Cette étape est suivie par l'ajout d'un polyester diacide sur le composé polyamide obtenu dans un premier temps, afin d'obtenir un composé polyamide soluble dans les huiles et apte à les gélifier.

L'invention a pour objet également les gels obtenus à partir de ces polyamides gélifiants. Ces gels se caractérisent par leur texture agréable au toucher, leur transparence, leur faible teneur en polyamide gélifiant, leur capacité de dispersion à des températures inférieures à 85°C, leur caractère thermoréversible et rhéofluidifiant et la possibilité d'être composé à 100% de produits d'origine naturelle.

Dans le cas de la gélification d'huiles cosmétiques, le polyamide gélifiant doit répondre à différents critères précis. Pour des raisons de coût, lors de son utilisation dans un produit cosmétique, le polyamide gélifiant doit tout d'abord être capable de gélifier la phase organique à une concentration faible, c'est-à-dire, de préférence, inférieure à 10% massique. De plus, un aspect important pour une application cosmétique est la sensation au toucher du produit. En effet, l'incorporation du gélifiant ne doit pas induire un effet granuleux trop marqué sur le produit cosmétique.

Il est donc recherché de disposer de composés gélifiants qui possèdent ces propriétés.

La demande WO98/17705 décrit la préparation de polyamides à terminaisons ester capables de gélifier des huiles, par un procédé en une étape. Les polyamides synthétisés sont capables de gélifier le tétradécane. Cependant, les polyamides obtenus ne sont pas de nature biosourcée et ne présentent donc pas l'origine naturelle désirée de la présente invention. De plus, les polymères obtenus ne peuvent gélifier qu'un nombre restreint d'huiles, et nécessitent la mise en oeuvre de concentrations élevées pour obtenir des gels forts, ce qui entraine des coûts de fabrication importants pour une formulation cosmétique.

Il est donc recherché de trouver des alternatives permettant de gélifier des huiles plus couramment utilisées, telle que le caprylic / capric triglycéride, ou les huiles végétales, avec des concentrations plus faibles.

On a maintenant trouvé que de nouveaux dérivés polyamides, préparés à partir de polyester d'origine naturelle, étaient particulièrement adaptés pour la gélification d'huiles végétales et organiques, et permettaient d'obtenir un gel ayant les qualités recherchées, notamment, pour une application cosmétique et/ou pharmaceutique.

On a également trouvé que les polyamides selon l'invention présentaient une facilité de mise en oeuvre dans les formulations cosmétiques et permettaient de leur conférer une texture onctueuse.

Par « d'origine naturelle », on entend un produit partiellement ou entièrement préparé à partir de matières naturelle (animale ou végétale) modifiées ou non chimiquement. Un tel produit peut être également qualifié de « biosourcé » ou « d'origine biosourcée », ces termes étant couramment utilisés dans le domaine des polymères.

Les produits biosourcés ou d'origine biosourcée sont généralement issus de matière végétale comme les arbres ou les plantes, par exemple la canne à sucre, la betterave, le tournesol, le palmier, le ricin et autres, ou de matière animale comme les graisses.

La teneur en contenu d'origine naturelle du polyamide, exprimée en pourcentage pondéral, peut être calculée, notamment, selon la norme NF ISO 16128-2.

Les diacides d'origine naturelle et/ou biosourcés peuvent être, par exemple, choisis parmi :
- L'acide 2,5-furandicarboxylique (C₆H₅O₄), obtenu par bio-fermentation du fructose par exemple ;
- L'acide itaconique (C₅H₆O₄), obtenu à partir du fructose par exemple ;
- L'acide succinique (C₄H₆O₄), obtenu à partir de glucose fermenté par E. Coli par exemple ;
- L'acide glutarique (C₅H₈O₄), obtenu à partir de sucre par exemple ;
- L'acide adipique (C₆H₁₀O₄), obtenu à partir de l'acide saccharique par exemple ;
- L'acide heptanedioique (C₇H₁₂O₄), obtenu à partir de l'huile de ricin par exemple ;
- L'acide azélaïque (C₉H₁₆O₄), obtenu à partir de l'acide oléïque par exemple ;
- L'acide sébacique (C₁₀H₁₈O₄), obtenu à partir de l'huile de ricin par exemple;
- L'acide undécanedioïque (C₁₁H₂₀O₄), obtenu à partir de l'huile de ricin par exemple;
- L'acide dodécanedioïque (C₁₂H₂₂O₄), obtenu à partir de bio-fermentation de l'acide laurique (coco) par exemple;
- L'acide brassylique (C₁₃H₂₄O₄), obtenu à partir de l'acide erucique (colza) par exemple;
- L'acide tetradécanedioïque (C₁₄H₂₆O₄), obtenu par bio-fermentation de l'acide myristique (coco) par exemple;
- L'acide hexadécanedioïque (C₁₆H₃₀O₄), obtenu par bio-fermentation de l'acide palmitique (palme) par exemple;
- L'acide octadécanedioïque (C₁₈H₃₄O₄), obtenu par bio-fermentation de l'acide stéarique par exemple;
- L'acide eicosanedioïque (C₂₀H₃₈O₄), obtenu par bio-fermentation de l'acide arachidique (colza) par exemple ;
- L'acide docosanedioïque (C₂₀H₃₈O₄), obtenu par métathèse de l'acide undécylénique (ricin) par exemple ;
- Les dimères d'acides gras (comportant 36 atomes de carbones notamment), obtenu à partir d'acides insaturés, tel que par exemple l'acide oléique ;
- Les polyesters diacides (polyester comportant des fonctions acides en bout de chaînes), obtenu à partir de dérivés d'huile biosourcée.

Les amines d'origine naturelle et/ou biosourcées peuvent, notamment, être obtenues par amination des acides dicarboxyliques cités précédemment, par exemple :
- Le butanediamine (C₄H₁₂N₂), obtenue par amination de l'acide succinique ;
- L'hexaméthylène diamine (C₆H₁₆N₂), obtenue par amination de l'acide adipique ;
- Les dimères diamines gras (comportant 36 atomes de carbones notamment), obtenus par amination des dimères d'acides gras.

Avantageusement, les nouveaux dérivés polyamides selon l'invention peuvent être préparés par un procédé de synthèse comprenant une étape de formation d'un précurseur, et une étape d'ajout d'un polyester diacide, apportant la solubilité dans les huiles au composé final.

L'invention concerne donc des composés polyamides de formule (1) 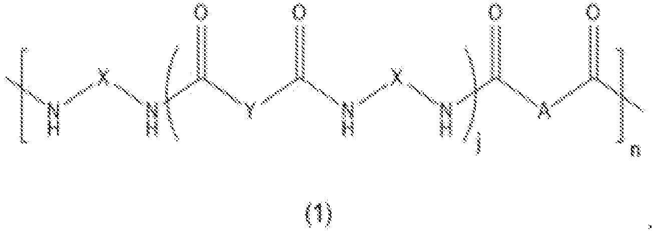 dans laquelle : 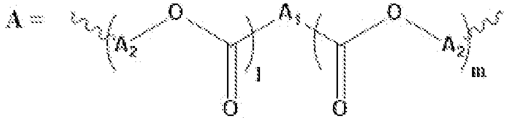
- A₁ représente un radical alkyl, linéaire ou ramifié, comprenant de 2 à 40 atomes de carbone, de préférence de 2 à 12, et en particulier de 2 à 6, ledit radical comprenant éventuellement une ou plusieurs insaturations, étant éventuellement interrompu par au moins un hétéroatome choisi parmi O, N et S, et étant éventuellement substitué par au moins un substituant -OAlk, Alk représentant un groupe alkyle comprenant de 1 à 10 atomes de carbone ;
- A₂ représente un radical alkyl, linéaire ou ramifié, comprenant de 2 à 30 atomes de carbone, de préférence de 5 à 22, notamment 5 à 18, ledit radical comprenant éventuellement une ou plusieurs insaturations, et étant éventuellement substitué par au moins un substituant -OAlk, Alk représentant un groupe alkyle comprenant de 1 à 10 atomes de carbone ;
- l et m représentent, indépendamment l'un de l'autre, un nombre entier de 1 à 20, de préférence de 1 à 10, la somme l+m étant, de préférence, de 2 à 20 ;
- n est un nombre entier de 1 à 20, de préférence de 1 à 8 ;
- j est un nombre entier de 1 à 10, de préférence de 1 à 3 ;
- X représente un radical alkyl, linéaire ou ramifié, comprenant de 2 à 60 atomes de carbone, de préférence 16 à 45 atomes de carbone, ledit radical comprenant éventuellement une ou plusieurs insaturations ;
- Y représente un radical alkyl, linéaire ou ramifié, comprenant de 3 à 35 atomes de carbone, de préférence 6 à 18 atomes de carbone, ledit radical comprenant éventuellement une ou plusieurs insaturations, ou un hétérocycle ayant 3 à 10 membres et comprenant 1 ou 2 hétéroatome(s) choisi(s) parmi O, N et S.

De préférence, A₁ représente un radical alkyl linéaire comprenant de 2 à 12, de préférence de 2 à 6, en particulier 3 atomes de carbone, ledit radical comprenant éventuellement une ou plusieurs insaturations.

De préférence, A₂ représente un radical alkyl, linéaire ou ramifié, comprenant de de 5 à 18 atomes de carbone, ledit radical comprenant éventuellement une ou plusieurs insaturations, et préférentiellement un groupe de formule

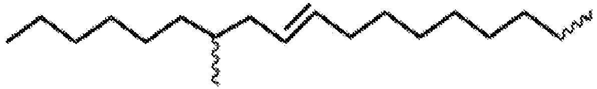

Avantageusement, la somme l+m a une valeur moyenne de 7, 11 ou 15.

Dans les formules A et A₂ ci-dessus, le signe 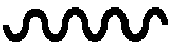 représente une liaison du segment considéré avec la chaine principale.

Des composés polyamides de formule (1) préférés sont ceux dans laquelle :
- A₁ représente un radical alkyl linéaire comprenant 3 atomes de carbone;
- A₂ représente un radical alkyl ramifié comprenant 17 atomes de carbone;
- l et m représentent, indépendamment l'un de l'autre, un nombre entier de 3 à 4, la somme l+m étant, de préférence, de 6 à 8;
- n est un nombre entier de 1 à 8, de préférence de 1 à 3;
- j est un nombre entier de 1 à 10, de préférence de 1 à 3 ;
- X représente un radical alkyl ramifié comprenant 36 atomes de carbone;
- Y représente un radical alkyl linéaire comprenant 8 atomes de carbone.

Dans la présente description, et sauf stipulation contraire, les gammes de valeur indiquées s'entendent bornes incluses.

L'invention concerne également un procédé de préparation de polyamides gélifiants. La préparation du polyamide gélifiant a pour objectif de modifier chimiquement un polyester, soluble dans les huiles, notamment végétales, afin d'y incorporer des groupements amide permettant ainsi d'apporter les propriétés de gélification souhaitées.

Selon l'invention, ledit procédé de préparation de polyamides gélifiants de formule (1) 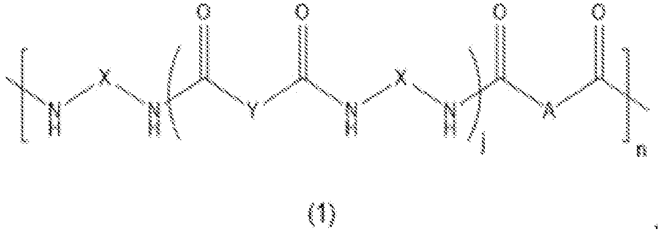 comprend les étapes suivantes :
1) La préparation d'un polyamide diamine par fonctionnalisation d'un diacide de formule (2) par une diamine de formule (3) pour obtenir un polyamide diamine de formule (4) : 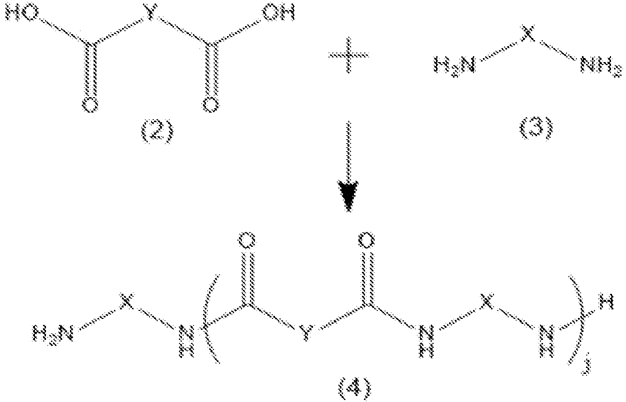 dans lesquelles :
   - X représente un radical alkyl, linéaire ou ramifié, comprenant de 2 à 60 atomes de carbone, de préférence 16 à 45 atomes de carbone, ledit radical comprenant éventuellement une ou plusieurs insaturations ;
   - Y représente un radical alkyl, linéaire ou ramifié, comprenant de 3 à 35 atomes de carbone, de préférence 6 à 18 atomes de carbone, ledit radical comprenant éventuellement une ou plusieurs insaturations ou un hétérocycle ayant 3 à 10 membres et comprenant 1 ou 2 hétéroatome(s) choisi(s) parmi O, N et S
   - j est un nombre entier de 1 à 10, de préférence de 1 à 3 ;
2) La modification d'un polyester diacide de formule (5) 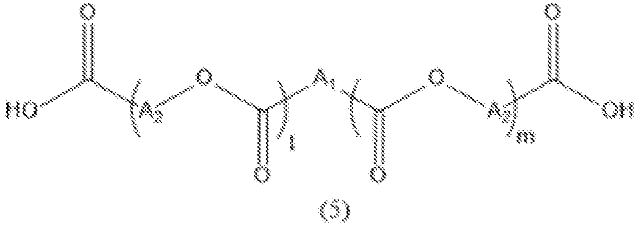 par ajout du polyamide diamine de formule (4) 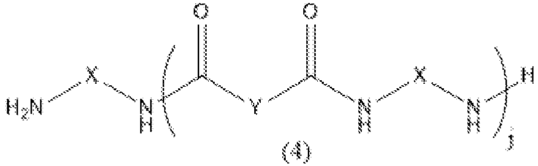 conduisant au polyamide de formule (1) ayant des propriétés gélifiantes, dans lesquelles : 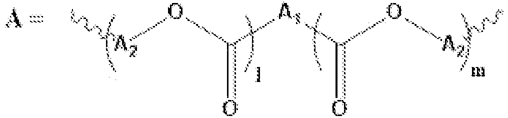
   - A₁ représente un radical alkyl, linéaire ou ramifié, comprenant de 2 à 40 atomes de carbone, de préférence de 2 à 12, et en particulier de 2 à 6, ledit radical comprenant éventuellement une ou plusieurs insaturations, étant éventuellement interrompu par au moins un hétéroatome choisi parmi O, N et S, et étant éventuellement substitué par au moins un substituant -OAlk, Alk représentant un groupe alkyle comprenant de 1 à 10 atomes de carbone ;
   - A₂ représente un radical alkyl, linéaire ou ramifié, comprenant de 2 à 30 atomes de carbone, de préférence de 5 à 22, ledit radical comprenant éventuellement une ou plusieurs insaturations, et étant éventuellement substitué par au moins un substituant
   - OAlk, Alk représentant un groupe alkyle comprenant de 1 à 10 atomes de carbone ;
   - l et m représentent, indépendamment l'un de l'autre, un nombre entier de 1 à 20, de préférence de 1 à 10, la somme l+m étant, de préférence, de 2 à 20 ;
   - n est un nombre entier de 1 à 20, de préférence de 1 à 8 ;
   - X, Y, j sont tels que définis précédemment.

Le polyamide diamine de formule (4) est indifféremment dénommé, dans la description, « précurseur diamine » ou « précurseur fonctionnalisé diamine ».

De manière optionnelle, le procédé selon l'invention peut comprendre, en outre une étape additionnelle comprenant la terminaison des chaînes du composé polyamide de formule (1) par ajout d'un composé de terminaison alcool de formule (6), conduisant à un composé polyamide de formule (7) : 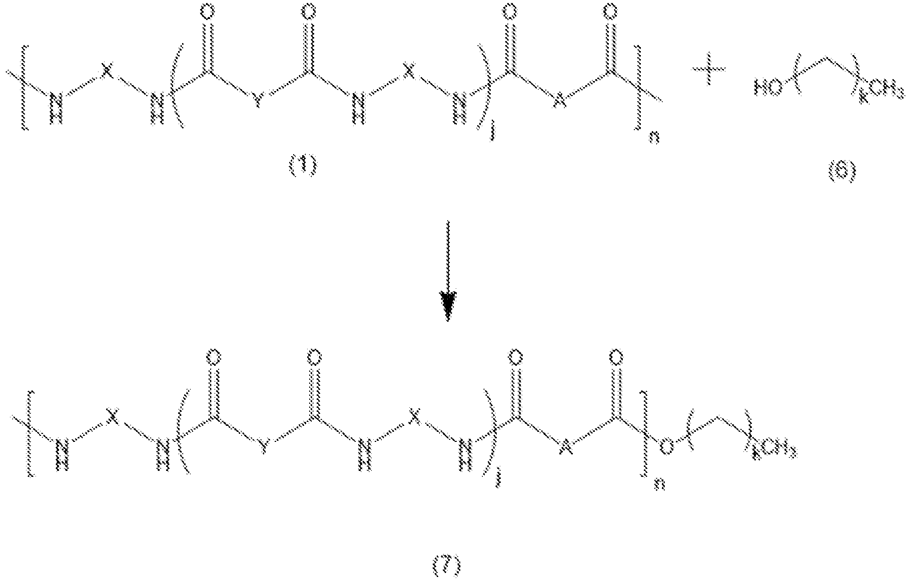 dans lesquelles : 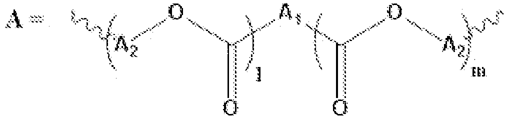
- X, Y, A, A₁, A₂, j, n sont tels que définis précédemment ;
- k est un nombre entier de 2 à 60, de préférence de 8 à 40.

Avantageusement, le composé polyamide de formule (7) présente une solubilité améliorée dans les huiles végétales.

Le composé polyamide de formule (7) et son utilisation comme gélifiant représentent également des aspects de l'invention.

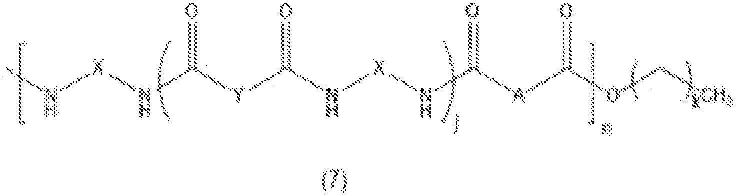

Tous les aspects préférés relatifs à A, A₁, A₂, X, Y, l, m, n, k et j mentionnés plus haut pour les composés polyamides de formule (1) s'appliquent également aux composés polyamides de formule (7) et au procédé de préparation selon l'invention.

Le polyester diacide de formule (5) 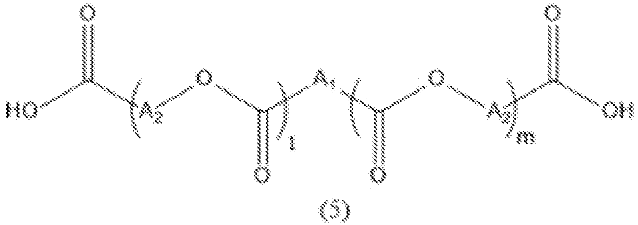 dans laquelle A₁, A₂, l et m sont tels que définis ci-dessus, est disponible commercialement sous le nom de PRICA^{®} (ITERG), ou peut être préparé par condensation d'un diacide de formule (8) avec un dérivé ester hydroxylé ou acide gras hydroxylé de formule (9) selon un protocole dérivé de la demande WO2014044809 :

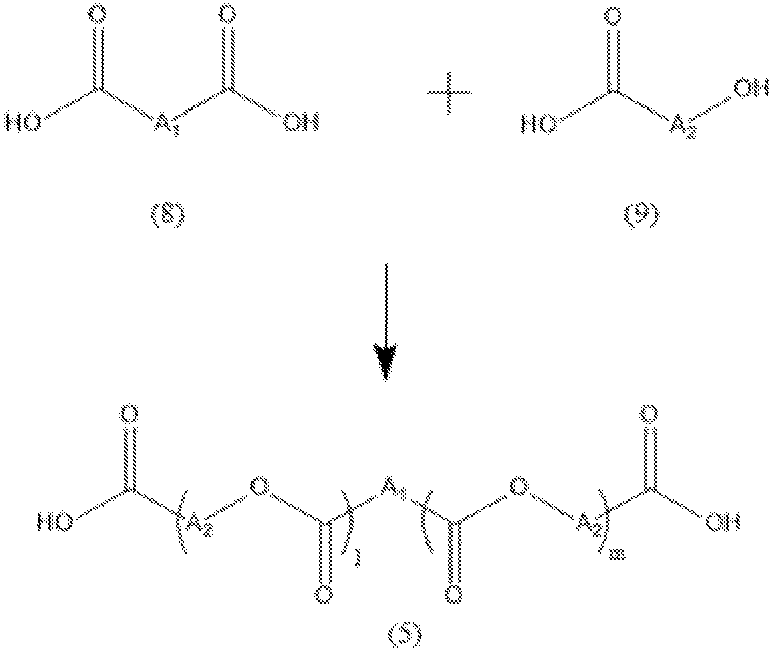

La 1^{ère} étape du procédé de préparation du composé polyamide gélifiant (1), à savoir l'étape de formation d'un précurseur diamine (4) par fonctionnalisation d'un diacide (2) par une diamine (3), permet la fabrication du bloc gélifiant. Cette étape a, de préférence, une durée comprise entre 1h et 24h, de préférence entre 4 h et 16h. La réaction est réalisée, de préférence, à une température comprise entre 100°C et 240°C, notamment entre 130°C et 160°C. Cette étape est réalisée, de préférence, dans un système étanche préalablement séché et placé sous vide.

De préférence, la 1^{ère} étape est effectuée par mélange d'un équivalent de diacide (2) et d'une quantité de diamine (3) comprise entre 1 et 4 équivalents. La quantité d'équivalent(s) de diamine (3) mise en oeuvre peut être calculée, par exemple, d'après l'indice d'amine de celle-ci.

Généralement, les équivalents sont calculés d'après les taux d'acide et d'amine fournis par les fabricants de matières premières dans le CoA (Certificate of Analysis) du produit ou sont déterminés par dosage.

Le diacide de formule (2) peut être choisi, par exemple, parmi les diacides aliphatiques en C₄-C₁₂, tels que l'acide sébacique, l'acide adipique, l'acide succinique ou les diacides comprenant un hétérocycle ayant 3 à 10 membres et comprenant 1 ou 2 héteroatome(s) choisi(s) parmi O, N et S, tel que l'acide furandicarboxylique (FDCA).

La diamine de formule (3) peut comprendre, par exemple, un radical alkyle comprenant 30 à 40 atomes de carbone.

Un diacide de formule (2) préféré est l'acide sébacique et une diamine de formule (3) préférée est un dimère diamine comportant en moyenne 36 carbones, commercialisée par CRODA sous le nom de Priamine^{®}.

La 2^{ème} étape du procédé selon l'invention (modification du polyamide de polyester) a, de préférence, une durée comprise entre 30 min et 24 h, notamment entre 2 h et 10 h. La réaction peut être effectuée, par exemple, à une température comprise entre 100°C et 240°C, de préférence entre 130°C et 160°C, par ajout de polyester diacide dans un réacteur contenant le précurseur formé lors de l'étape 1, préalablement chauffé et agité à la température de l'étape 2.

Le ratio entre le polyester diacide (5) et le précurseur diamine (4) est un des facteurs qui influe sur les capacités de gélification du produit final.

Le ratio entre le précurseur diamine (4) et le diacide (5) est choisi de telle sorte que le composé (1) ne présente plus de fonctions amines en bout de chaine, mais uniquement des fonctions acide carboxylique ou alcool.

On utilisera de préférence une quantité de précurseur diamine (4) comprise entre 0,2 et 1 équivalent (eq), et plus particulièrement entre 0,4 et 0,7 équivalent (eq) pour 1 équivalent de polyester diacide (5).

Avantageusement, la 2^{ème} étape est effectuée en ajoutant 1 équivalent de polyester diacide (5) sur une quantité de précurseur diamine (4) comprise entre 0,2 et 1 équivalent, à une température comprise entre 100 et 240°C, de préférence entre 130 et 150°C.

A la fin de la 2^{ème} étape, le polyamide gélifiant est formé, et la récupération du polyamide gélifiant peut être effectuée, par exemple, en le faisant couler à une température supérieure à 100°C du réacteur vers un récipient adapté.

Le procédé selon l'invention présente l'avantage de consister en une polymérisation en masse, utilisant des composés biosourcés et/ou d'origine naturelle. Les polyamides gélifiants ainsi obtenus permettent l'obtention de gels qui peuvent être 100% biosourcés lorsque le polyamide gélifiant est solubilisé dans une huile végétale.

De plus, la réalisation de la synthèse en deux étapes permet la fabrication de petits blocs polyamides apportant les propriétés de gélification, puis le greffage de ces groupes sur des polyesters qui apportent ensuite les propriétés de solubilité du composé dans les huiles d'origine végétale.

L'invention concerne également l'utilisation du polyamide gélifiant selon l'invention, ou obtenu selon le procédé de l'invention, comme agent gélifiant de milieux organiques, notamment les huiles végétales. En particulier, l'invention concerne également l'utilisation du polyamide gélifiant selon l'invention, ou obtenu selon le procédé de l'invention, comme agent gélifiant d'huiles choisies, par exemple, parmi les huiles végétales ou d'origine naturelle, telles que le caprylic/capric triglycéride, helianthus annuus seed oil, ethylhexyl palmitate, castor oil, coconut oil, glycine soja oil, Polyglyceryl-4 Oleate (and) Glyceryl Olivate (and) Hydrogenated Rapeseed Alcohol, Isopropyl myristate, dicaprylyl carbonate, ethyl oleate, prunus domestica seed oil, prunus amygdalus dulcis oil, sesamum indicum seed oil, simmondsia chinensis seed oil, olea europaea fruit oil, vitis vinifera seed oil, punica granatum seed oil, ricinus communis seed oil, triticum vulgare germ oil, octyldodecanol, coco-caprylate, et corylus avellana seed oil (dénomination INCI).

Le polyamide gélifiant selon l'invention peut également former un gel clair et transparent avec les huiles apolaires si elles sont mélangées à des huiles plus polaires, comme par exemple, un mélange contenant jusqu'à 70% de C15-19 Alkane ou 70% de Squalane avec 30% de Caprylic/capric tryglyceride.

Avantageusement, le polyamide gélifiant de formule (1) ou (7), tel que décrit plus haut, permet d'obtenir un gel présentant une combinaison de propriétés avantageuses, à savoir : des propriétés de force en gel, des propriétés de transparence, une texture agréable au toucher et des charges uniformément réparties dans le gel grâce à la capacité suspensive dudit polyamide vis-à-vis desdites charges dans le gel, ces propriétés étant, de plus, obtenues en utilisant un composé biosourcé.

Par « capacité suspensive », on entend la capacité à maintenir des solides en suspension dans le milieu sans qu'il se produise de précipitation.

Avantageusement, le gel obtenu à partir du polyamide gélifiant de formule (1) ou (7), tel que décrit plus haut, est rhéofluidifiant (sa viscosité dynamique baisse lorsque le taux de cisaillement augmente) et thixotrope (sa viscosité apparente diminue lorsqu'il est soumis à un taux de cisaillement constant, et revient à sa valeur initiale lorsqu'on arrête le cisaillement).

Le polyamide gélifiant selon l'invention peut également être utilisé pour former des billes d'huile gélifiée en suspension dans un milieu aqueux. Pour cela, le polyamide gélifiant sera préalablement dispersé dans une huile d'intérêt, puis des billes seront obtenus par ajout de l'huile gélifiée dans la phase aqueuse, soit par dispersion mécanique, soit par des procédés de microfluidique (billes millimétriques). Le mélange pourra éventuellement être stabilisé soit par l'ajout d'agents tensioactifs pour obtenir une suspension, soit par ajout d'agents de coacervation. Les avantages apportés par le gélifiant consistent en des propriétés mécaniques des billes accrues, ainsi qu'une texture agréable et une plus grande stabilité des billes en suspension dans la phase aqueuse.

L'invention concerne également un procédé de gélification d'au moins une huile choisie, par exemple, parmi les huiles végétales et les mélanges d'huiles apolaires et d'huiles plus polaires, en particulier d'origine naturelle, dans lequel un polyamide gélifiant de formule (1) ou (7) tel que décrit plus haut, selon l'invention, ou obtenu par le procédé de préparation décrit plus haut, est ajouté à ladite ou auxdites huile(s), ou à une composition la ou les contenant.

L'invention concerne également les gels contenant ou formés à partir desdits polyamides gélifiants.

En particulier, les gels comprenant le polyamide gélifiant selon l'invention, ou obtenu selon le procédé de l'invention, et au moins une huile acceptable sur le plan pharmaceutique ou cosmétique sont un objet de l'invention.

Selon un aspect préféré, le polyamide gélifiant selon l'invention, ou obtenu selon le procédé de l'invention permet de former, par exemple, un gel rhéofluidifiant et thixotrope à une teneur de 5% massique dans du caprylic/capric triglycéride.

Le polyamide gélifiant selon l'invention, ou obtenu selon le procédé de l'invention, comme agent gélifiant forme des gels dans le caprylic/capric triglycéride dont la viscosité augmente avec la concentration dudit gélifiant.

Le gel formé avec le polyamide gélifiant selon l'invention, ou obtenu selon le procédé de l'invention peut atteindre, notamment, un optimum de viscosité 4 h seulement après sa préparation, et rester la même 48h plus tard.

Les polyamides gélifiants selon l'invention permettent de former des gels visqueux contenant jusqu'à 5% d'éthanol.

Également, le polyamide gélifiant selon l'invention, ou obtenu selon le procédé de l'invention permet de gélifier des huiles et de stabiliser des mélanges contenant jusqu'à 4% de cires avec les cires suivantes : cire d'abeille, C10-18 triglycérides, behenyl alcohol, glyceryl stéarate, synthetic wax, pentaerythrityl distearate, copernica cerifera wax, cetearyl alcohol, cetyl alcohol, rice bran wax, sunflower wax, hydrogenated cocoglycerides, glyceryl dibehenate, C18-36 acid triglycéride. (Dénomination INCI).

Le polyamide gélifiant permet également de gélifier des huiles et de stabiliser des mélanges contenant jusqu'à 4% en poids de beurres avec, par exemple, les beurres suivants : beurre de karité, beurre d'abricot, beurre de coco.

L'invention concerne également les gels contenant ou formés à partir du polyamide gélifiant selon l'invention, ou obtenu selon le procédé de l'invention. En effet, les polyamides gélifiants de formule (1) ou (7) permettent de préparer facilement des gels clairs huileux totalement naturels.

Les polyamides gélifiants selon l'invention permettent également de former des gels clairs avec les filtres solaires tels que ethylhexylmethoxycinnamate, ethylhexyl salicylate, homosalate, octocrylene.

La teneur massique du polyamide gélifiant dans ledit gel est, de préférence, de 0,5 à 10%, notamment de 3 à 8%.

L'invention a également pour objet les compositions comprenant un polyamide gélifiant de formule (1) ou (7) tel que décrit ci-dessus ou obtenus selon le procédé de l'invention, ou comprenant les gels contenant ou formés à partir desdits polyamides gélifiants, en particulier les compositions pharmaceutiques ou cosmétiques.

Lesdites compositions pharmaceutiques ou cosmétiques peuvent comprendre des ingrédients actifs et des véhicules et additifs pharmaceutiquement acceptables ou acceptables en cosmétique.

La teneur massique du polyamide gélifiant dans une composition cosmétique selon l'invention peut être, par exemple, comprise entre 0,5% et 10%, de préférence de 1 à 10%, notamment de 3 à 8%. Les polyamides gélifiants selon l'invention, ou obtenus selon le procédé de l'invention, peuvent donc être associés en cosmétique avec des huiles polaires, apolaires, végétales, ou des parfums.

Ils sont, en particulier, compatibles avec les filtres solaires organiques et minéraux, les pigments et les nacres, les actifs huileux couramment utilisés en cosmétique, les beurres et les cires cosmétiques ou les émulsionnants.

Le polyamide gélifiant permet également de gélifier des huiles et de stabiliser des mélanges contenant jusqu'à 10% d'agent tensioactif, par exemple : PEG-20 sorbitan oleate, PEG-20 glyceryl triisostearate, polyglyceryl-4 oleate, polyglyceryl-4-stearate.

Le polyamide gélifiant permet également de gélifier des huiles et de stabiliser des mélanges contenant jusqu'à 5% d'agent à suspendre tels que silica, titanium dioxide, clay, mica, Luffa Cylindrica Fruit, cellulose acetate et vaccinum microcarpon fruit powder.

Les polyamides gélifiants décrits ci-dessus peuvent être incorporés dans des formulations galéniques variées anhydres telles que gel anhydre, gel vaporisable, gel pompable, gel nacré, gel teinté, baume à lèvres, stick à lèvre, gel exfoliant, gel parfumé, gelée solaire, gel démaquillant huileux.

Ils peuvent également être incorporés dans des formulations de type émulsion, telle que, par exemple, une émulsion eau dans huile, une émulsion huile dans eau, une émulsion solaire, un fond de teint eau dans huile, un fond de teint huile dans eau.

Le polyamide gélifiant selon l'invention, ou obtenu selon le procédé de l'invention, est particulièrement adapté à une utilisation dans une composition cosmétique.

L'invention est illustrée par les exemples suivants.

Dans les exemples, le polyester diacide PRICA 2405^{®} est un polyester commercialisé par l'ITERG, l'Institut des Corps Gras. La Priamine 1075@ est une diamine commercialisée par CRODA. L'acide sébacique provient de ARKEMA (biosourcé) ou de Sigma Aldrich. Le Labrafac CC^{®} (Nom INCI : Caprylic/Capric Triglycéride, également dénommé ci-après « CCT ») provient de chez Gattefossé. L'huile de tournesol (Nom INCI : helanthius annuus seed oil) provient de IES Labo.

Les préparations 1 à 9 décrivent la synthèse du précurseur fonctionnalisé diamine de formule (4), qui constitue le bloc gélifiant du produit final.

Les exemples 1 à 9 et 11 à 13 concernent les composés polyamides gélifiants de formule (1). L'exemple 10 est un exemple comparatif. L'exemple 14 décrit la préparation d'un composé polyamide gélifiant de formule (7).

Les exemples 15 à 23 concernent l'étude des propriétés des composés polyamides gélifiants de formule (1) et les compositions les contenant.

### Exemples

Préparation 1 : Préparation du précurseur fonctionnalisé diamine (4) à partir de 1 équivalent de diacide (2) pour 2 équivalents de diamine (3).

Le précurseur fonctionnalisé diamine est préparé à partir de l'acide sébacique et de la Priamine 1075^{®}.

32,01g de Priamine 10750 et 6,00g d'acide sébacique sont introduits dans un ballon de 100mL. Le mélange réactionnel est laissé sous agitation magnétique à 140°C pendant 24h sous vide. La réaction est suivie par infrarouge, notamment par accroissement et stabilisation du pic lié aux fonction amides (C=O, 1636 cm⁻¹). La chromatographie d'exclusion stérique (CES) en étalonnage polystyrène indique une masse molaire moyenne en nombre (Mn) de 600 g/mol.

Préparation 2 : Préparation du précurseur fonctionnalisé diamine (4) à partir de 1 équivalent de diacide (2) pour 2,5 équivalents de diamine (3).

Le précurseur fonctionnalisé diamine est préparé à partir de l'acide sébacique et de la Priamine 1075^{®}.

40,07g de Priamine 10750 et 6,00g d'acide sébacique sont introduits dans un ballon de 100mL. Le mélange réactionnel est laissé sous agitation magnétique à 140°C pendant 24h sous vide. La réaction est suivie par infrarouge, notamment par accroissement et stabilisation du pic lié aux fonctions amides (C=O, 1636 cm⁻¹). La chromatographie d'exclusion stérique (CES) en étalonnage polystyrène indique une masse molaire moyenne en nombre (Mn) de 590 g/mol.

Un essai de gélification a été effectué. Le gel est réalisé à une concentration de 5% massique du produit obtenu dans le CCT (Caprylic/Capric Triglycéride). 0,5g du produit final sont introduits et complétés par 9,51g de CCT dans un pilulier de 20mL. Le pilulier serti est placé dans un agitateur horizontal préchauffé à 100°C et agité pendant 30 min. Après 24h à température ambiante, un gel opaque blanc est obtenu. Le greffage du bloc gélifiant (4) sur le polyester diacide (5), parfaitement soluble dans les huiles, est donc nécessaire pour permettre la transparence des gels.

Préparation 3 : Préparation du précurseur fonctionnalisé diamine (4) à partir de 1 équivalent de diacide (2) pour 1 équivalent de diamine (3).

Le précurseur fonctionnalisé diamine est préparé à partir de l'acide sébacique et de la Priamine 1075^{®}.

26,67g de Priamine 10750 et 10,00g d'acide sébacique sont introduits dans un ballon de 100mL. Le mélange réactionnel est laissé sous agitation mécanique à 140°C pendant 6h sous vide. 2,65g de Priamine 1075^{®} sont ajoutés au milieu réactionnel comme agent de terminaison, puis la réaction est poursuivie pendant 6h sous vide. La réaction est suivie par infrarouge, notamment par accroissement et stabilisation du pic lié aux fonctions amides (C=O, 1636 cm⁻¹). La chromatographie d'exclusion stérique (CES) en étalonnage polystyrène indique une masse molaire moyenne en nombre (Mn) de 1980 g/mol.

Préparation 4 :(SNO19019) : Préparation du précurseur fonctionnalisé diamine (4) à partir de 1 équivalent de diacide (2) pour 2,5 équivalents de diamine (3).

Le précurseur fonctionnalisé diamine est préparé à partir de l'acide sébacique et de la Priamine 1075^{®}.

508,50g de Priamine 10750 et 76,26g d'acide sébacique sont introduits dans un réacteur de 1L. Le mélange réactionnel est laissé sous agitation mécanique à 140°C pendant 12h sous vide. La réaction est suivie par infrarouge, notamment par accroissement et stabilisation du pic lié aux fonctions amides (C=O, 1636 cm⁻¹). La chromatographie d'exclusion stérique (CES) en étalonnage polystyrène indique une masse molaire moyenne en nombre (Mn) de 540 g/mol.

Un essai de gélification a été effectué. Le gel est réalisé à une concentration de 5% massique du produit obtenu dans le CCT (Caprylic/Capric Triglycéride). 0,49g du produit final sont introduits et complétés par 9,50g de CCT dans un pilulier de 20mL. Le pilulier serti est placé dans un agitateur horizontal préchauffé à 80°C et agité pendant 30 min. Après 24h à température ambiante, un gel opaque blanc est obtenu. Le greffage du bloc gélifiant (4) sur le polyester diacide (5), parfaitement soluble dans les huiles, est donc nécessaire pour permettre la transparence des gels.

Préparation 5 (SNO19043) : Préparation du précurseur fonctionnalisé diamine (4) à partir de 1 équivalent de diacide (2) pour 2,5 équivalents de diamine (3).

Le précurseur fonctionnalisé diamine est préparé à partir de l'acide sébacique et de la Priamine 1075^{®}.

464,38g de Priamine 10750 et 69,0g d'acide sébacique sont introduits dans un réacteur de 1L. Le mélange réactionnel est laissé sous agitation mécanique à 140°C pendant 12h sous vide. La réaction est suivie par infrarouge, notamment par accroissement et stabilisation du pic lié aux fonctions amides (C=O, 1636 cm⁻¹). La chromatographie d'exclusion stérique (CES) en étalonnage polystyrène indique une masse molaire moyenne en nombre (Mn) de 540 g/mol.

Préparation 6 : Préparation du précurseur fonctionnalisé diamine (4) à partir de 1 équivalent de diacide (2) pour 2,5 équivalents de diamine (3).

Le précurseur fonctionnalisé diamine est préparé à partir de l'acide succinique (Sigma Aldrich) et de la Priamine 1075^{®}.

34,29g de Priamine 10750 et 2,998g d'acide succinique sont introduits dans un ballon de 100mL. Le mélange réactionnel est laissé sous agitation magnétique à 180°C pendant 1h sous flux d'azote puis 3h sous vide. La réaction est suivie par infrarouge, notamment par accroissement et stabilisation du pic lié aux fonctions amides (C=O, 1636 cm⁻¹). La chromatographie d'exclusion stérique (CES) en étalonnage polystyrène indique une masse molaire moyenne en nombre (Mn) de 540 g/mol.

Préparation 7 : Préparation du précurseur fonctionnalisé diamine (4) à partir de 1 équivalent de diacide (2) pour 2,5 équivalents de diamine (3).

Le précurseur fonctionnalisé diamine est préparé à partir de l'acide adipique (Sigma Aldrich) et de la Priamine 1075^{®}.

27,78g de Priamine 10750 et 3,01g d'acide adipique sont introduits dans un ballon de 100mL. Le mélange réactionnel est laissé sous agitation magnétique à 140°C pendant 24h sous vide. La réaction est suivie par infrarouge, notamment par accroissement et stabilisation du pic lié aux fonctions amides (C=O, 1636 cm⁻¹). La chromatographie d'exclusion stérique (CES) en étalonnage polystyrène indique une masse molaire moyenne en nombre (Mn) de 570 g/mol.

Préparation 8 : Préparation du précurseur fonctionnalisé diamine (4) à partir de 1 équivalent de diacide (2) pour 2,5 équivalents de diamine (3).

Le précurseur fonctionnalisé diamine est préparé à partir de l'acide itaconique (Sigma Aldrich) et de la Priamine 1075^{®}.

31,10g de Priamine 1075^{®} et 3,01g d'acide itaconique sont introduits dans un ballon de 100mL. Le mélange réactionnel est laissé sous agitation magnétique à 140°C pendant 7h sous vide puis à 180°C pendant 3h sous vide. La réaction est suivie par infrarouge, notamment par accroissement et stabilisation du pic lié aux fonctions amides (C=O, 1636 cm⁻¹). La chromatographie d'exclusion stérique (CES) en étalonnage polystyrène indique une masse molaire moyenne en nombre (Mn) de 570 g/mol.

Préparation 9 : Préparation du précurseur fonctionnalisé diamine (4) à partir de 1 équivalent de diacide (2) pour 2,5 équivalents de diamine (3).

Le précurseur fonctionnalisé diamine est préparé à partir de l'acide furandicarboxylique (FDCA) (Sigma Aldrich) et de la Priamine 1075^{®}.

Dans un ballon de 100mL, 3,23g de FDCA sont dispersés à chaud (100°C) dans 28g de butanol, puis 28g de Priamine 1075^{®} sont ajoutés. Le mélange est ensuite mis à 140°C sous vide durant 24h. Le butanol est distillé et récupéré à l'aide d'un montage type Dean-Stark. Le mélange est ensuite placé à 200°C sous vide durant 8h. La réaction est suivie par infrarouge, notamment par accroissement et stabilisation du pic lié aux fonctions amides (C=O, 1636 cm⁻¹). La chromatographie d'exclusion stérique (CES) en étalonnage polystyrène indique une masse molaire moyenne en nombre (Mn) de 680 g/mol.

### Exemple 1 : Préparation d'un polyamide (1) à partir d'un polyester diacides (PRICA^{®}) (5) et du précurseur fonctionnalisé diamine (4) (préparation 1)

24,00g de PRICA 2405^{®} et 6,28g du précurseur synthétisé dans la préparation 1 sont introduits dans un ballon de 100mL. Le mélange réactionnel est laissé sous agitation magnétique à 140°C pendant 24h sous vide. La réaction est suivie par infrarouge, notamment par accroissement et stabilisation du pic lié aux fonctions amides (C=O, 1636 cm⁻¹). La chromatographie d'exclusion stérique (CES) en étalonnage polystyrène indique une masse molaire moyenne en nombre (Mn) de 5900 g/mol.

Un essai de gélification a été effectué. Le gel est réalisé à une concentration de 5% massique du produit obtenu dans le CCT. 0,3334g d'un prélèvement effectué au bout de 4h de réaction sont introduits et complétés jusqu'à 6,667g avec du CCT dans un pilulier de 10mL. 0.337g du produit final (24h de réaction) sont introduits et complétés jusqu'à 6,740g avec du CCT dans un pilulier de 10mL. Les piluliers sertis sont placés dans un agitateur horizontal préchauffé à 100°C et agités pendant 30 min. Après 24h à température ambiante, des gels translucides sont obtenus.

### Exemple 2 : Préparation d'un polyamide (1) à partir d'un polyester diacide (PRICA^{®}) (5) et du précurseur fonctionnalisé diamine (4) (préparation 2)

24,03g de PRICA 2405^{®} et 5,84g du précurseur synthétisé dans la préparation 2 sont introduits dans un ballon de 100mL. Le mélange réactionnel est laissé sous agitation magnétique à 140°C pendant 24h sous vide. La réaction est suivie par infrarouge, notamment par accroissement et stabilisation du pic lié aux fonctions amides (C=O, 1636 cm⁻¹). La chromatographie d'exclusion stérique (CES) en étalonnage polystyrène indique une masse molaire moyenne en nombre (Mn) de 6100 g/mol.

Un essai de gélification a été effectué. Le gel est réalisé à une concentration de 5% massique du produit obtenu dans le CCT. 5,00g du produit final et 95,01g de CCT sont introduits dans un bécher de 150mL. Le bécher est placé dans un bain marie préchauffé à 108°C et l'agitation mécanique est lancée à 200 rpm lorsque la température du contenu du bécher atteint 95°C puis est augmentée à 300 rpm lorsque le contenu du bécher atteint 98°C. L'agitation est maintenue 30min à 100 ± 2°C. Le contenu du bécher est versé dans deux pots en plastique. Après 24h, les gels obtenus sont transparents.

### Exemple 3 : Préparation d'un polyamide (1) à partir d'un polyester diacides (PRICA^{®}) (5) et du précurseur fonctionnalisé diamine (4) (préparation 3)

24,01g de PRICA 2405^{®} et 7,46g du précurseur synthétisé dans la préparation 3 sont introduits dans un ballon de 100mL. Le mélange réactionnel est laissé sous agitation magnétique à 140°C pendant 24h sous vide. La réaction est suivie par infrarouge, notamment par accroissement et stabilisation du pic lié aux fonctions amide (C=O, 1636 cm⁻¹).

Un essai de gélification a été effectué. Le gel est réalisé à une concentration de 5% massique du produit obtenu dans le CCT. 0,355g du produit final (24h de réaction) sont introduits et complétés jusqu'à 7,079g avec du CCT dans un pilulier de 10mL. Le pilulier serti est placé dans un agitateur horizontal préchauffé à 100°C et agités pendant 30 min. Après 24h à température ambiante, le gel obtenu est trouble.

### Exemple 4 (SNO19025) : Préparation d'un polyamide (1) à partir d'un polyester diacide (PRICA^{®}) (5) et du précurseur fonctionnalisé diamine (4) (préparation 4).

751,96g de PRICA 2405^{®} et 146,53g du précurseur synthétisé dans la préparation 4 sont introduits dans un réacteur de 1,7L. Le mélange réactionnel est laissé sous agitation mécanique à 140°C pendant 4,5h sous vide. La réaction est suivie par infrarouge, notamment par accroissement et stabilisation du pic lié aux fonctions amides (C=O, 1637 cm⁻¹). La chromatographie d'exclusion stérique (CES) en étalonnage polystyrène indique une masse molaire moyenne en nombre (Mn) de 2500 g/mol (Mp 7060 g/mol).

Des essais de gélification ont été effectués. Les gels sont réalisés aux concentrations de 4%, 5% et 10% massique du produit obtenu dans le CCT. Le produit final est introduit et complété par du CCT dans un pilulier de 20mL. Le pilulier serti est placé dans un agitateur horizontal préchauffé à 80°C et agité pendant 30 min. Les gels obtenus sont transparents après 24h.

### Exemple 5 (SNO19046) : Préparation d'un polyamide (1) à partir d'un polyester diacide (PRICA^{®}) (5) et du précurseur fonctionnalisé diamine (4) (préparation 5).

523,07g de PRICA 2405^{®} et 103,01g du précurseur synthétisé dans la préparation 5 sont introduits dans un réacteur de 1L. Le mélange réactionnel est laissé sous agitation mécanique à 140°C pendant 4,5h sous vide. La réaction est suivie par infrarouge, notamment par accroissement et stabilisation du pic lié aux fonctions amide (C=O, 1637 cm⁻¹). La chromatographie d'exclusion stérique (CES) en étalonnage polystyrène indique une masse molaire moyenne en nombre (Mn) de 2800 g/mol (Mp 7300 g/mol).

Des essais de gélification ont été effectués. Les gels sont réalisés aux concentrations de 4% et 10% massique du produit obtenu dans le CCT. Le produit final est introduit et complété par CCT dans un pilulier de 20mL. Le pilulier serti est placé dans un agitateur horizontal préchauffé à 80°C et agité pendant 30 min. Les gels obtenus sont transparents après 24h.

### Exemple 6 (SNO19024) : Préparation d'un polyamide (1) à partir d'un polyester diacide (PRICA^{®}) (5) et du précurseur fonctionnalisé diamine (4) (préparation 4).

12,51g de PRICA 2405^{®} et 2,34g du précurseur synthétisé dans la préparation 4 sont introduits dans un ballon de 100mL. Le mélange réactionnel est laissé sous agitation magnétique à 140°C pendant 4h sous vide. La réaction est suivie par infrarouge, notamment par accroissement et stabilisation du pic lié aux fonctions amide (C=O, 1636 cm⁻¹). La chromatographie d'exclusion stérique (CES) en étalonnage polystyrène indique une masse molaire moyenne en nombre (Mn) de 3300 g/mol (Mp 8100 g/mol).

### Exemple 7 (SNO19014) : Préparation d'un polyamide (1) à partir d'un polyester diacide (PRICA^{®}) (5) et du précurseur fonctionnalisé diamine (4) (préparation 2).

30,0g de PRICA 2405^{®} et 7,50g du précurseur synthétisé dans la préparation 2 sont introduits dans un ballon de 100mL. Le mélange réactionnel est laissé sous agitation magnétique à 140°C pendant 4h sous vide. La réaction est suivie par infrarouge, notamment par accroissement et stabilisation du pic lié aux fonctions amides (C=O, 1636 cm⁻¹). La chromatographie d'exclusion stérique (CES) en étalonnage polystyrène indique une masse molaire moyenne en nombre de 2800 g/mol (Mp 7300 g/mol).

### Exemple 8 : Préparation d'un polyamide (1) à partir d'un polyester diacide (PRICA^{®}) (5) et du précurseur fonctionnalisé diamine (4)

24,01g de PRICA 2405^{®} et 3,11g du précurseur de la préparation 2 sont introduits dans un ballon de 100mL. Le mélange réactionnel est laissé sous agitation magnétique à 140°C pendant 4h sous vide. La réaction est suivie par infrarouge, notamment par accroissement et stabilisation du pic lié aux fonctions amide (C=O, 1636 cm⁻¹). La chromatographie d'exclusion stérique (CES) en étalonnage polystyrène indique une masse molaire moyenne en nombre de 5600 g/mol.

Un essai de gélification a été effectué. Le gel est réalisé à une concentration de 5% massique du produit obtenu dans le CCT. 0,341g du produit final (4h de réaction) sont complétés par du CCT jusqu'à 6,84g dans un pilulier de 10mL. Le pilulier serti est placé dans un agitateur horizontal préchauffé à 80°C et agités pendant 30 min. Après 24h à température ambiante, un gel parfaitement transparent est obtenu.

### Exemple 9 : Préparation d'un polyamide (1) à partir d'un polyester diacide (PRICA^{®}) (5) et du précurseur fonctionnalisé diamine (4).

Dans un ballon de 100mL, 18,42g de PRICA 2405^{®} et 8,02g du précurseur de la préparation 2 sont introduits. Le mélange réactionnel est laissé sous agitation magnétique à 140°C pendant 24h sous vide. La réaction est suivie par infrarouge, notamment par accroissement et stabilisation du pic lié aux fonctions amide (C=O, 1636 cm⁻¹). La chromatographie d'exclusion stérique (CES) en étalonnage polystyrène indique une masse molaire moyenne en nombre de 5600 g/mol.

Un essai de gélification a été effectué. Le gel est réalisé à une concentration de 5% massique du produit obtenu dans le CCT. 0,338g du produit final sont complétés par du CCT jusqu'à 6,86g dans un pilulier de 10mL. Le pilulier serti est placé dans un agitateur horizontal préchauffé à 80°C et agités pendant 30 min. Après 24h à température ambiante, un gel trouble est obtenu.

### Exemple 10 : Préparation du polyamide à partir d'un polyester diacide (PRICA^{®}) (5) et d'une diamine (Priamine^{®}) (3)

Dans un ballon de 100mL, 25,10g de PRICA 2405^{®} et 2,62g de Priamine 10750 sont introduits. Le mélange réactionnel est laissé sous agitation magnétique à 140°C pendant 4h sous vide. La réaction est suivie par infrarouge, notamment par accroissement et stabilisation du pic lié aux fonctions amide (C=O, 1636 cm⁻¹).

Un essai de gélification a été effectué. Le gel est réalisé à une concentration de 5% massique du produit obtenu dans le CCT. 0,50g du produit final (4h de réaction) sont introduits et complétés jusqu'à 10,01g avec du CCT dans un pilulier de 20mL. Le pilulier serti est placé dans un agitateur horizontal préchauffé à 80°C et agités pendant 30 min. Après 24h à température ambiante, la solution est transparente mais n'est pas gélifiée. L'utilisation d'un bloc gélifiant (4) est donc nécessaire pour l'obtention d'un polyamide gélifiant les huiles.

### Exemple 11: Essais de gélification de différentes huiles effectuées avec les polyamides gélifiants synthétisés (5% en masse).

Le polyamide (1) utilisé pour les essais de gélification est issu de 0,55 équivalent du précurseur gélifiant (4), constitué d'un équivalent d'acide sébacique (2) pour 2,5 équivalents de Priamine 1075^{®} (3), et d'un équivalent de PRICA 2405^{®} (5).

Les résultats sont rapportés dans le tableau 1 ci-dessous. Les huiles sont identifiées par leur dénomination INCI.

Le gélifiant a été solubilisé dans l'huile à 80°C sous agitation.

La force des gels a été évaluée de la manière suivante : la transparence des gels est évaluée qualitativement, par observation d'un pilulier contenant du gel. S'il est possible de lire des caractères écrits de façon très nette à travers le pilulier, alors le gélifiant est considéré comme formant des gels transparents. Le gel est considéré comme fort s'il ne s'écoule pas lorsque le pilulier est renversé et faible si la surface de l'échantillon se déforme ou que le gel s'écoule.

**Tableau 1**

| **INCI** | **Gel** | **Transparence** |
|---|---|---|
| HELIANTHUS ANNUUS SEED OIL | fort | Translucide |
| GLYCINE SOJA OIL | fort | Translucide |
| SESAMUM INDICUM SEED OIL | fort | Translucide |
| PRUNUS AMYGDALUS DULCIS OIL | fort | Translucide |
| SIMMONDSIA CHINENSIS SEED OIL | fort | Translucide |
| OLEA EUROPAEA FRUIT OIL | fort | Translucide |
| VITIS VINIFERA SEED OIL | fort | Translucide |
| PUNICA GRANATUM SEED OIL | fort | Translucide |
| CORYLUS AVELLANA SEED OIL | fort | Translucide |
| PRUNUS DOMESTICA SEED OIL | fort | Translucide |
| RICINUS COMMUNIS SEED OIL | fort | Transparent |
| TRITICUM VULGARE GERM OIL | fort | Translucide |
| OCTYLDODECANOL | fort | Transparent |
| ETHYLHEXYL PALMITATE | fort | Transparent |
| COCO-CAPRYLATE | faible | Transparent |
| CAPRYLIC/CAPRIC TRIGLYCERIDE | fort | Transparent |

### Exemple 12 : Mesure de la viscosité de gels réalisés dans le Caprylic/Capric Triglycéride avec les polyamides des exemples 1,2,4 et 5 à une concentration de 5% en masse.

Les mesures de viscosité ont été réalisées avec un viscosimètre TA Instruments AR1500ex par mesure de rhéologie en oscillation, à une température de 25°C, à une contrainte d'oscillation de 1 Pa et à une fréquence de 1Hz.

**Tableau 2**

| Echantillons | SOV18028 | SOV19014 | SOV19007 | SOV19016-2 |
|---|---|---|---|---|
| | Exemple 1 | Exemple 2 | Exemple 3 | Exemple 9 |
| Viscosité (Pa.s) | 11,4 | 2,7 | 0,5 | 38,8 |

### Exemple 13: Préparation d'un polyamide (1) à partir d'un polyester diacide (PRICA^{®}) (5) et du précurseur fonctionnalisé diamine (4) (préparation 7).

20,00g de PRICA 2405^{®} (5) et 7,55g du précurseur (4) synthétisé dans la préparation 7 sont introduits dans un ballon de 100mL. Le mélange réactionnel est laissé sous agitation magnétique à 140°C pendant 4h sous vide. La réaction est suivie par infrarouge, notamment par accroissement et stabilisation du pic lié aux fonctions amide (C=O, 1636 cm⁻¹).

Un essai de gélification a été effectué. Le gel est réalisé à une concentration de 5% massique du produit obtenu dans le CCT. 0,50g du produit final est introduit dans un pilulier et est complété jusqu'à 10,01g par du CCT. Le pilulier serti est placé dans agitateur horizontal chauffé à 80°C pendant 30min. Après 24h, le mélange est gélifié et est parfaitement transparent.

### Exemple 14: Terminaison des chaînes du composé gélifiant (1) à l'aide d'un composé hydroxylé (6).

20,00g du composé gélifiant (1) (identique à l'exemple 2) et 1,00g d'alcool cétylique (Evonik) sont introduits dans un premier ballon de 100mL. Dans un second ballon de 100mL, jouant le rôle de contrôle, 15,39g du composé gélifiant (1) sont introduits. Les mélanges réactionnels sont laissés sous agitation magnétique à 200°C pendant 1h30 sous vide. Les réactions sont suivies par infrarouge, notamment par accroissement et stabilisation du pic lié à la fonction ester (C=O, 1730 cm⁻¹).

Des essais de gélification ont été effectués. Les gels sont réalisés à une concentration de 5% massique du produit obtenu dans l'huile de tournesol (INCI : Helianthus Annuus seed oil). 0,50g du produit final terminé par l'alcool cétylique et 9,50g d'huile de tournesol sont introduits dans un pilulier. 0,51g du produit final contrôle et 9,51g d'huile de tournesol sont introduits dans un second pilulier. Les piluliers sertis sont placés dans un agitateur horizontal chauffé à 80°C pendant 30min. Après 24h, les mélanges sont gélifiés et le pilulier contenant le produit terminé par l'acide cétylique est transparent tandis que le pilulier contenant le produit témoin est translucide.

### Exemple 15 : comparaison des propriétés du composé polyamide (1) de l'exemple 4 (SNO19025) avec des composés gélifiants commercialement disponibles

Chaque gélifiant a été mis en solution dans l'huile Caprylic/capric triglycéride (CCT) en suivant le protocole proposé par le fournisseur.

Chaque mélange est ensuite évalué le lendemain en ce qui concerne :
- ses caractéristiques organoleptiques, son aspect visuel, sa transparence, sa couleur, son homogénéité, et
- ses propriétés sensorielles, en touchant directement une noisette de gel sur la main.

Les résultats rapportés dans le tableau 3 ci-dessous permettent de comparer la teneur en contenu d'origine naturelle (naturalité) du gélifiant, sa capacité à former un gel, l'aspect du gel, sa transparence ainsi que sa sensorialité (texture) lorsque cela a été évalué.

**Tableau 3**

| **INCI** | **Teneur en contenu d'origine naturelle** | **% de gélifiant** | **Mode opératoire** | **Aspect** | **Texture** |
|---|---|---|---|---|---|
| Composé (1) exemple 4 SNO19025 | 100% | 4 | 30 min défloculeuse 80°C 300 rpm | Gel épais transparent Mémoire de forme * | Toucher onctueux fini poudré |
| Silica dimethyl sililate | 0% | 2,6 | 15min défloculeuse 80°C 700rpm | Gel transparent Dépôt fluide | Toucher crissant sec |
| Helianthus Annuus Seed oil, Caprylic/Capric Triglycéride, Styrene/ Butadiene Copolymer | 0% (Polymère) | 13 | 15min défloculeuse 60°C 700rpm | Gel liquide transparent | |
| Polyurethane-79 | 0% | 5 | 30 min défloculeuse 95°C 700RPM | Gel épais transparent | Toucher léger sec |
| Caprylic/Capric Triglycéride, Stearalkonium Bentonite, Propylene Carbonate | Partiellement biosourcé | 12 | 15min défloculeuse 70°C 700 rpm | Gel opaque verdâtre liquide | |
| Polyamide-8 | Partiellement biosourcé | 5 | 25min défloculeuse 80°C 700RPM+ 20min Rotor Stator 15000 rpm | Gel blanc opaque épais | |
| Dextrin Palmitate | 100% | 5 | 30 min défloculeuse 80°C 700 rpm | Gel opaque épais | Toucher poudré sec |
| Trihydroxystearin | 100% | 3 | 65min défloculeuse 50°C 700 rpm | Gel blanc opaque fluide | |
| Castor oil /IPDI copolymer | 91% | 5 | 30 min défloculeuse 100°C 700 rpm | Gel épais transparent | Toucher doux, léger |

| | | | | | |
|---|---|---|---|---|---|
| * Mémoire de forme : La texture du gel se reforme après trituration | | | | | |

Les résultats montrent que le composé (1) selon l'invention permet de combiner les propriétés de transparence, toucher non granuleux, en utilisant un polymère entièrement biosourcé et à l'état de solution à faible température (à 80°C).

Les gélifiants non biosourcés (2^{ème}, 3^{ème} et 4^{ème} gélifiants testés) présentent les désavantages suivants :
- Les dérivés de silice nécessitent un pourcentage élevé pour leur mise en oeuvre et présente un sensoriel peu agréable jugé « crissant ».
- Les « styrene copolymer » ne sont pas adapté à l'application recherchée car la mise en oeuvre du polymère pur nécessitant beaucoup d'énergie, les produits disponibles sont toujours en solution.
- Le polyurethane-79 nécessite une température élevée (95°C) pour une mise en oeuvre optimale et les gels obtenus manquent de stabilité au cours du temps.

Parmi les alternatives d'origine naturelle existantes sur le marché, aucune ne permet de former des gels transparents dans les conditions testées.

### Exemple 16 : Propriétés gélifiantes vis-à-vis de différentes huiles

Le polyamide gélifiant de l'exemple 7 (SNO19014) a été introduit à une concentration massique de 2% dans diverses huiles, puis agité à 80°C durant 30 min. Les résultats sont rapportés dans le tableau 4 ci-dessous.

Chaque mélange est évalué visuellement le lendemain sur son aspect gel ou non (le pillulier est renversé, si le produit est un gel alors le produit ne s'écoule pas), sa transparence ou sa turbidité à l'aide d'une feuille quadrillée disposée à l'arrière du pilulier.

**Tableau 4**

| **INCI** | **Obtention d'un gel** | **Aspect** |
|---|---|---|
| Ethylhexyl palmitate | oui | clair |
| Caprylic/capric triglycéride | oui | clair |
| Helianthus anuus (Sunflower) oil | oui | clair |
| Castor oil | oui | clair |
| Coconut oil | oui | clair |
| Glycine soja oil | oui | trouble |
| Ethylhexyl methoxy cinnamate | oui | clair |
| Ethylhexyl Methoxycrylene | oui | opaque |
| Ethylhexyl salicylate | oui | clair |
| Homosalate | oui | clair |
| Octocrylene | oui | opaque |
| Myristate d'isopropyl | oui | clair |
| Coco caprylate | oui | clair |
| Dicaprylyl carbonate | oui | clair |
| Dimethicone | non | trouble |
| Coco-Caprylate/Caprate | oui | clair |
| Ethyl oleate | oui | clair |
| Prunus domestica | oui | trouble |
| Corylus avellana seed oil | oui | trouble |

Les résultats montrent que le polyamide gélifiant selon l'invention permet de former un gel transparent dans 13 des huiles testées et de former un gel non transparent dans 5 des huiles testées.

Le polyamide gélifiant selon l'invention permet de gélifier notamment les huiles polaires : les huiles végétales, les esters et la plupart des filtres solaires organiques.

### Exemple 17 : étude de la capacité gélifiante dans l'huile Caprylic/capric triglycéride

Le polyamide gélifiant de l'exemple 5 (SNO19046) a été introduit à des concentrations massiques croissantes (de 0,5% à 10% massique) dans de l'huile Caprylic/capric triglycéride contenant des nacres, puis agité à 80°C durant 30 min à l'aide d'une défloculeuse à 300rpm.

Chaque mélange est évalué visuellement le lendemain en ce qui concerne ses caractéristiques organoleptiques, son aspect, sa transparence, sa couleur et son homogénéité.

La viscosité est mesurée à l'aide d'un viscosimètre Brookfield LVDVII+ équipé d'un module Helipath à la vitesse de 10 tours par minute. La mesure est prise après une minute de trituration. Les résultats sont rapportés dans les tableaux 5 et 6.

On a également mesuré la capacité suspensive vis-à-vis de nacres, ainsi que la capacité du mélange gélifié à être pulvérisé (sprayabilité).

Par « capacité suspensive », on entend la capacité à maintenir des solides en suspension dans le milieu sans qu'il se produise de précipitation dans le temps. Cette capacité est évaluée visuellement juste après préparation, puis confirmée le lendemain de la préparation du gel, la quantité de nacres présente au fond du pilulier étant alors visuellement évaluée.

**Tableau 5**

| % de composé gélifiant (exemple 5) | 0,5 | 1 | 2 | 4 |
|---|---|---|---|---|
| Viscosité 24h (Pa.s) | 0,50 | 1,00 | 1,75 | 3,60 |
| Aspect | gel clair transparent | gel très clair et homogène | gel très clair et homogène | gel très clair légèrement jaune |
| Capacité suspensive (0,05% nacre) | immédiate | immédiate | immédiate | immédiate |
| Sprayabilité | oui | oui | oui | oui |

**Tableau 6**

| % de composé gélifiant (exemple 5) | 6 | 8 | 10 |
|---|---|---|---|
| Viscosité 24h (Pa.s) | 6,00 | 8,10 | 9,70 |
| Aspect | gel très clair légèrement jaune | gel très clair légèrement jaune | gel très transparent |
| Capacité suspensive(0,05% nacre) | immédiate | immédiate | immédiate |
| Sprayabilité | oui | oui avec jet unidirectionnel | difficile à pulvériser |

La capacité suspensive du gel formé par l'invention est immédiate, dès 60°C durant le protocole et ce dès 0.5% de polyamide.

La galénique du gel formé permet d'être pulvérisé à l'aide d'un spray aisément jusqu'à au moins 6% de polyamide gélifiant.

La prise du gel dans le temps a ensuite été évaluée pour le gel à 6% de polyamide gélifiant dans l'huile Caprylic/capric triglycéride. La viscosité a été mesurée à l'aide d'un viscosimètre brookfield LVDVII+ et d'un module Helipath à la vitesse de 10 rpm, la mesure étant prise après une minute. Les mesures sont ensuite effectuées 30 min 1h, 2h, 4h, 6h, 8h, 24h, 30h et 48h après préparation. La viscosité mesurée est de 6,6 Pa.s après 4h, 6,6 Pa.s après 8h et 7 Pa.s après 48h, montrant que le gel se forme rapidement.

### Exemple 18

Le gel contenant 4% de l'exemple 4 dans le Caprylic/capric triglycéride est combiné à de l'éthanol à différents pourcentages pour en vérifier sa compatibilité. L'aspect et la viscosité du gel est évalué.

**Tableau 7**

| % d'éthanol | 0 | 1 | 2 | 5 | 10 |
|---|---|---|---|---|---|
| Viscosité (Pa.s) | 3,6 | 2,3 | 1,3 | 0,65 | 0,35 |
| Aspect | Clair, gel homogène | Clair, gel homogène | Clair, gel homogène | Clair, gel homogène | Clair, gel homogène, très fluide |

La viscosité est mesurée, à l'aide d'un viscosimètre Brookfield LVDVII+ et d'un module Helipath à la vitesse de 10 RPM, la mesure est prise après une minute. Elle est fortement impactée dès l'ajout de 1% d'éthanol au mélange.

### Exemple 19 : compatibilité avec des cires et des beurres

La compatibilité du gélifiant avec des cires et des beurres est évaluée. Un gel contenant 4% de polyamide de l'exemple 4 (SNO19025) est testé en combinaison avec différentes cires. Chaque essai est préparé de la manière suivante : une référence contenant la cire à évaluer à 4% dans du Caprylic/Capric triglycéride, un test contenant la cire à évaluer, 4% de gélifiant de l'exemple 4 et de l'huile Caprylic/Capric triglycérides. Les essais sont préparés sur 10g et chauffés à 80°C durant 30 min sous agitation mécanique.

Chaque mélange est évalué visuellement le lendemain sur ses caractéristiques organoleptiques, son aspect visuel, sa transparence à l'aide d'un papier quadrillé placé à l'arrière du pilulier, sa couleur et son homogénéité.

Les résultats sont rapportés dans le tableau 8.

**Tableau 8**

| **INCI** | **Aspect gel sans gélifiant** | **Aspect gel avec gélifiant** |
|---|---|---|
| Hydrogenated CocoGlycerides | liquide clair homogène | gel clair homogène |
| C10-18 Triglycérides | liquide clair homogène | gel clair homogène |
| Shea Butter | liquide clair homogène | gel clair homogène |
| Cera alba | solide opaque homogène | solide opaque homogène |
| Behenyl alcohol | gel opaque homogène | solide opaque homogène |
| Cetyl alcohol | liquide clair homogène | gel clair homogène |
| Glyceryl stéarate | gel opaque homogène | gel opaque légèrement jaune homogène |
| Myristyl miristate | liquide clair homogène | gel clair homogène |
| Parrafin wax | solide blanc homogène | gel beige homogène |
| Cetyl stearyl alcohol | liquide clair homogène | gel clair homogène |
| Oryza sativa oil | liquide opaque homogène | gel faible opaque homogène |
| Helianthus annuus seed cera | gel épais opaque | gel épais opaque homogène |
| C18-36 Acid Triglycéride | gel épais opaque | gel épais opaque homogène |

Les résultats montrent que la présence du polyamide gélifiant selon l'invention permet d'obtenir un mélange plus visqueux, plus homogène et plus lisse.

### Exemple 20 : compatibilité avec des tensioactifs

La compatibilité avec les tensioactifs a été testée de la même manière que pour les cires et beurres dans l'exemple 22. Les résultats sont rapportés dans le tableau 9.

**Tableau 9**

| INCI | aspect gel sans gélifiant de l'invention | aspect gel avec gélifiant de l'invention |
|---|---|---|
| PEG-40 sorbitan peroleate | liquide limpide et homogène | gel clair homogène |
| PEG-20 Glyceryl Triisostearate | liquide limpide et homogène | gel clair homogène |
| Polyglyceryl-3 Oleate | liquide limpide et homogène | gel clair homogène |
| Polyglyceryl-4 Isostearate | liquide limpide et homogène | gel clair homogène |

Les résultats montrent que, pour toutes les matières testées, le mélange est gélifié par le polyamide de l'invention et est homogène. Ces ingrédients sont bien compatibles.

### Exemple 21 : Pouvoir de suspension avec différents agents de charge

La capacité suspensive du polymère gélifiant de l'exemple 4 a été évaluée avec différents agents de charge : pigments, poudre et agent exfoliant.

Ces agents de charge sont testés chacun à une concentration massique de 5% dans le même gel de base contenant 4% en masse de gélifiant de l'exemple 4 (SNO19025) dans l'huile Caprylic/capric triglycéride.

Le polyamide est ajouté à l'huile, chauffé à 80°C sous une agitation de 300 rpm à l'aide d'une pâle défloculeuse durant 30 min, le mélange est ensuite refroidi et les charges sont ajoutées sous la même agitation à une température de 40°C.

Chaque mélange est ensuite évalué le lendemain en ce qui concerne :
- ses caractéristiques organoleptiques, son aspect visuel, sa transparence, sa couleur et son homogénéité,
- sa viscosité mesurée à l'aide d'un viscosimètre Brookfield LVDVII+ et d'un module Helipath à la vitesse de 10 rpm, la mesure étant prise après une minute.

**Tableau 10**

| **INCI** | **aspect** | **viscosité (93) 10rpm 1 min (Pa.s)** |
|---|---|---|
| dioxide titane, silica (filtre UV) | gel fluide clair | 1,5 |
| dioxide titane (pigment) | gel bloc | 3,2 |
| sugar | gel avec dépôt de sucre | 3,3 |
| Mica (78-88%) and titanium dioxide (pigment) | gel nacré | 3,5 |
| Clay | gel noir | 3,2 |
| Volcanic Sand (sable) | gel avec gradient de grains | 2,58 |
| Luffa Cylindrica Fruit (agent exfoliant) | gel homogène | 4,93 |
| cellulose acetate (agent exfoliant) | gel homogène | 3,82 |
| Vaccinium macrocarpon fruitpowder (agent exfoliant) | gel épais | 4,40 |

Les résultats montrent que le gel à 4 % de polyamide gélifiant selon l'invention permet de suspendre immédiatement l'intégralité des charges lors de la fabrication. Le polyamide gélifiant selon l'invention peut donc être utilisé dans des formules cosmétiques contenant des charges et des poudres.

### Exemple 22 : étude de la transparence et de la viscosité

Les mesures ont été effectuées sur les gels obtenus avec les composés (1) des exemples 4 et 5 dans l'huile Caprylic/capric Triglycéride, à une concentration de 4% massique.

Les mesures ont été effectuées comme suit :
- Les mesures de viscosité ont été réalisées avec un viscosimètre TA Instruments AR1500ex par mesure de rhéologie en oscillation, à une température de 25°C, à une contrainte d'oscillation de 1 Pa et à une fréquence de 1Hz.
- La mesure de la transparence a été réalisé sur un gel de polymère (exemple 4 SNO19025, exemple 5 SNO19046 et Dextrin palmitate) dans une huile végétale (Caprylic/capric Triglycéride ou huile de tournesol) avec un turbidimètre Hanna Instruments HI 88713 à une température de 25°C et exprimée en unité de turbidité « NTU » prescrite par l'Environmental Protection Agency (Nephelometric Turbidity Unit).

A titre comparatif, on a mesuré la transparence sur le gel de Dextrin palmitate dans 4% massique de Caprylic/capric Triglycéride. Ce gel est opaque par observation à l'œil nu.

**Tableau 11**

| Exemple | Huile | Concentration % massique | Viscosité, Pa.s (oscillation, 25°C, 1Hz, 1Pa) | Transparence, NTU |
|---|---|---|---|---|
| Exemple 4 (SNO19025) | Caprylic/capric Triglycéride | 4 | 1,08 | 5 |
| | Caprylic/capric Triglycéride | 10 | 3,4 | 13 |
| | Helianthus annuus seed oil (Huile de tournesol) | 4 | 1,5 | 6 |
| Exemple 5 (SNO19046) | Caprylic/capric Triglycéride | 4 | 1,7 | 12 |
| | Caprylic/capric Triglycéride | 10 | 6,3 | 13 |
| - (contrôle) | Caprylic/capric Triglycéride | 0 | 0,02 | 0,1 |
| - (contrôle) | Helianthus annuus seed oil (Huile de tournesol) | 0 | 0,06 | 0,2 |
| Exemple comparatif Dextrin palmitate (Rheopearl KL 2) | Caprylic/capric Triglycéride | 4 | - | 2760 |

### Exemple 23: Incorporation du polyamide gélifiant (1) dans des formulations cosmétiques.

Le polyamide gélifiant obtenu dans l'exemple 2 a été incorporé au sein de formulations cosmétiques.

### 1) Gélification de l'huile de tournesol contenant un parfum à faible teneur (0,5%m)

Dans un bécher, 96,51g d'huile de tournesol et 3,00g du polyamide gélifiant (1) sont introduits, chauffés à 80°C sous agitation mécanique (300 rpm) pendant 20min. Le mélange homogène obtenu est refroidi jusqu'à 50°C et 0,49g de parfum sont ajoutés. Après 24h à température ambiante, le mélange est homogène, gélifié et possède une viscosité de 22 ,7 Pa.s.

### 2) Suspension de nacres dans l'huile de tournesol.

Dans un bécher, 96,60g d'huile de tournesol, 2,02g du polyamide gélifiant (1) et 1,00g de nacres sont introduits, chauffés à 80°C sous agitation mécanique (300 rpm) pendant 20min. Le mélange homogène obtenu est refroidi jusqu'à 50°C et 0,52g de parfum sont ajoutés. Après 24h à température ambiante, le mélange est homogène, les nacres sont uniformément réparties dans le milieu, et possède une viscosité de 13,5 Pa.s.

### 3) Suspension de billes d'exfoliant dans l'huile de tournesol.

Dans un bécher, 97,40g d'huile de tournesol, 2,00g du polyamide gélifiant (1) et 0,10g de billes d'exfoliant sont introduits et chauffés à 80°C sous agitation mécanique (300 rpm) pendant 20min. Le mélange homogène obtenu est refroidi jusqu'à 50°C et 0,52g de parfum sont ajoutés. Après 24h à température ambiante, le mélange est homogène, les billes sont uniformément réparties dans le milieu, et possède une viscosité de 14,5 Pa.s.

### 4) Suspension de dioxyde de titane dans l'huile de tournesol.

Dans un premier bécher, 52,56g d'huile de tournesol et 25,00g de dioxyde de titane sont introduits et dispersés à l'aide d'un mélangeur à haut cisaillement pendant 5 min à température ambiante. Dans un second bécher, 20,00g d'huile de tournesol et 2,00g du polyamide gélifiant (1) sont introduits et mélangés pendant 15 min à 80°C sous agitation mécanique (300 rpm). Ce bécher est introduit dans le premier bécher, préalablement chauffé à 80°C sous agitation mécanique (300 rpm), et l'agitation est maintenue 10 min supplémentaires. Le mélange homogène obtenu est refroidi jusqu'à 50°C et 0,50g de parfum sont ajoutés. Après 24h à température ambiante, le mélange est homogène, les particules de dioxyde de titane sont uniformément réparties dans le milieu, et possède une viscosité de 55 Pa.s.

### 5) Emulsion eau dans huile.

Dans un premier bécher, 72,03g d'huile de tournesol et 4,00g d'émulsionnant (Easynov) et 4,00g du polyamide gélifiant (1) sont introduits, chauffés à 80°C et mélangés à l'aide d'un agitateur mécanique (300 rpm). Dans un second bécher, 110,00g d'eau déminéralisée et 1,01g de sel (NaCl) sont introduits et mélangés pendant 5 min à 70°C sous agitation mécanique (300 rpm). Ce bécher est introduit dans le premier bécher au goutte à goutte et l'agitation est maintenue jusqu'à refroidissement de la solution à 50°C, où 0,50g de parfum sont ajoutés. Un mélange homogène est obtenu. Après 24h à température ambiante, le mélange est homogène et gélifié.

### 6) Gélification d'un parfum

Dans un bécher, 70,00g d'huile de tournesol et 10,00g du polyamide gélifiant (1) sont introduits, chauffés à 80°C sous agitation mécanique (300 rpm) pendant 20min. Le mélange homogène obtenu est refroidi jusqu'à 50°C et 19,94g de parfum sont ajoutés. L'agitation est maintenue pendant 2min. Après 24h à température ambiante, le mélange est homogène, gélifié et possède une viscosité de 65 Pa.s.

### 7) Gel antiâge transparent

On a préparé une formule complète contenant le polyamide gélifiant de l'exemple 4 :

**Tableau 12**

| **INCI** | **%** |
|---|---|
| Caprylic capric triglycérides | 88,3 |
| Composé de l'exemple 4 (SNO19025) | 6 |
| Simmondsia chinensis oil | 5 |
| parfum | 0,5 |
| dl-α-tocopheryl acetate | 0,2 |

Le gel est épais et onctueux, parfaitement transparent. Sa viscosité à J+1 est de 5,2 Pa.s. Le polyamide selon l'invention permet de former une texture facile à présenter en tube ou flacon, facile à étaler et douce au toucher.

### 8) Spray fluide nacré nourrissant visage

Une formule plus fluide contenant le polyamide gélifiant de l'exemple 4 est réalisée avec des nacres en suspensions.

**Tableau 13**

| **INCI** | **%** |
|---|---|
| Caprylic capric triglycérides | 93,5 |
| Composé de l'exemple 4 (SNO19025) | 1 |
| Simmondsia chinensis oil | 5 |
| parfum | 0,5 |
| mica, titanium dioxide | 0,5 |
| dl-α-tocopheryl acetate | 0,2 |

Cette formule est très fluide et les nacres sont maintenues en suspension à J+1.

Sa viscosité est inférieure à 0,5 Pa.s.

Le polyamide de l'exemple 4 permet donc de maintenir en suspension des charges nacrées à 0,5%. Cette capacité suspensive est efficace dès la mise en oeuvre du polymère, elle est immédiate après préparation.

### 9) Gel sublimant visage

Un autre gel nacré contenant le polyamide gélifiant de l'exemple 4, présentable en flacon ou en pot, a été préparé.

**Tableau 14**

| **INCI** | **%** |
|---|---|
| Ethylhexyl palmitate | 44,1 |
| Isopropyl Myristate | 50 |
| Mica, titanium dioxide | 0,2 |
| Composé de l'exemple 4 (SNO19025) | 5 |
| dl-α-tocopheryl acetate | 0,2 |
| Parfum | 0,5 |

Ce gel présente un sensoriel sec et poudré, le polyamide gélifiant de l'exemple 4 permet ici de former un gel de viscosité moyenne présentable en flacon et de maintenir les nacres en suspension de manière efficace.

Sa viscosité est de 1,7 Pa.s à J+1.

La texture de ce gel plutôt fluide se reforme après trituration tel un gel à mémoire de forme. Cela permet une application plutôt originale.

### 10) Baume nourrissant corporel

On a préparé un baume épais au toucher sec, contenant le polyamide de l'exemple 4.

**Tableau 15**

| **INCI** | **%** |
|---|---|
| Ethylhexyl palmitate | 34,1 |
| Isopropyl Myristate | 50 |
| Mica, titanium dioxide | 0,2 |
| Composé de l'exemple 4 (SNO19025) | 15 |
| dl-α-tocopheryl acetate | 0,2 |
| Parfum | 0,5 |

Ce gel solide présente un toucher doux et sec. Lors de la trituration, le gel se reforme, réagissant tel un gel à mémoire de forme. Le polyamide de l'invention permet de gélifier fortement le mélange et d'obtenir un gel solide et onctueux.

Ce pourcentage élevé de polyamide est facile à mettre en oeuvre. Les nacres sont bien maintenues en suspension et la viscosité est de 6,0 Pa.s à J+1.

### 11) Emulsion huile dans eau

On a préparé une émulsion à phase continue aqueuse contenant le polyamide de l'exemple 4.

**Tableau 16**

| **INCI** | **%** |
|---|---|
| Caprylic/capric triglycérides | 10 |
| C14-22 alcohols (and) C12-20 alkyl glucoside | 3 |
| Myristyl alcohol (and) myristyl glucoside | 0,5 |
| Composé de l'exemple 4 (SNO19025) | 5 |
| Water/Aqua | 72,2 |
| Propanediol | 2 |
| Xanthan gum | 0,2 |
| Glycerin | 3 |
| Pentylene glycol | 4 |
| Sodium Phytate | 0,1 |

La viscosité est plus forte pour la formule contenant le gélifiant :
Viscosité Référence = 1,5 Pa.s / Viscosité Formule avec le polyamide = 2,3 Pa.s

La centrifugation de la formule témoin présente un déphasage de quelques millimètres alors que la formule avec le polyamide de l'invention est stable et homogène.

La formule avec le polyamide gélifiant est donc plus stable que celle qui n'en contient pas. Le polyamide gélifiant permet donc de stabiliser les émulsions à phase continue aqueuse.

Le toucher de la crème qui contient le polymère est plus riche, onctueux et laisse un film homogène nourrissant après étalement sur la peau.

Sur la même base, une CC cream contenant 2% d'une nacre Sunshine Soft Bronze C84-6080^{®} (Sun Chemicals) a été formulée. A l'application, la couvrance de la teinte est améliorée en présence du polyamide gélifiant selon l'invention et l'étalement est plus précis. La formule contenant le polyamide est aussi plus stable dans le temps que la formule de référence qui ne contient pas le gélifiant.

### 12) Emulsion eau dans huile n°1

Une émulsion eau dans huile contenant le polyamide de l'exemple 4 est formulée avec un émulsionnant classiquement utilisé dans les formules de ce type.

**Tableau 17**

| **INCI** | **%** |
|---|---|
| Butyrospermum parkii Butter | 5 |
| Polyglyceryl-2-Dipolyhydroxystearate | 5 |
| Caprylic/Capric triglycéride | 20 |
| Composé de l'exemple 4 (SNO19025) | 4 |
| Tocopheryl acetate | 0,3 |
| Aqua | 54,5 |
| Magnésium sulfate | 0,7 |
| Glycerin | 5 |
| Pentylene glycol | 5 |
| Perfume | 0,5 |

L'émulsion réalisée avec le polyamide gélifiant de l'exemple 4 est plus épaisse que la formule de référence sans le polymère.

Référence : viscosité 6,4 Pa.s / Essai avec polymère : viscosité 24,2 Pa.s.

Les deux formules sont stables en centrifugation et l'émulsion s'est bien formée. L'émulsion avec le polyamide selon l'invention est plus riche et confortable. Le toucher de la crème qui contient le polyamide est plus riche, onctueux et laisse un film nourrissant sur la peau après étalement.

### 13) Emulsion eau dans huile n°2

Un autre type d'émulsion eau dans l'huile a été préparé.

**Tableau 18**

| **INCI** | **%** |
|---|---|
| Polyglyceryl-6 Polyhydroxystearate (and) Polyglyceryl-6 Polyricinoleate | 3,50 |
| Coco-Caprylate | 10,00 |
| Caprylic/capric triglycéride | 5,00 |
| Corylus avelanna oil | 5,00 |
| C10-18 Triglycérides | 5,00 |
| Composé de l'exemple 4 (SNO19025) | 6,00 |
| Aqua | 62,70 |
| Sodium chloride | 1,00 |
| Ethylhexylglycerin & Phenoxyethanol | 0,80 |
| parfum | 1,00 |

Cette émulsion est stable en centrifugation, elle est facile à prélever et présente un étalement onctueux. Elle laisse un film riche sur la peau. La formule contenant le polyamide de l'invention est plus stable que celle n'en contenant pas.

### 14) Fond de teint eau dans huile

En utilisant la base d'émulsion précédente, on a préparé un fond de teint eau dans huile contenant 6% du polyamide gélifiant de l'exemple 4.

**Tableau 19**

| **INCI** | **%** |
|---|---|
| Polyglyceryl-6 Polyhydroxystearate | 3,50 |
| (and) | |
| Polyglyceryl-6 Polyricinoleate | |
| Coco-Caprylate | 5,00 |
| Caprylic/capric triglycéride | 5,00 |
| Corylus avelanna oil | 5,00 |
| C10-18 Triglycérides | 5,00 |
| Composé de l'exemple 4 (SNO19025) | 6,00 |
| Aqua | 58,14 |
| Sodium chloride | 1,00 |
| Ethylhexylglycerin & Phenoxyethanol | 0,80 |
| parfum | 1,00 |
| iron oxyde white | 8 |
| iron oxyde yellow | 0,4 |
| iron oxyde red | 0,16 |
| iron oxyde black | 1 |

L'émulsion s'est bien formée, les pigments se sont intégrés de manière homogène à l'émulsion. La teinte est homogène et la formule est stable en centrifugation. La viscosité de cette crème est de 33,3 Pa.s.

La même émulsion réalisée sans le polyamide gélifiant a une viscosité de 17 Pa.s.

Le polyamide gélifiant de l'invention permet donc d'obtenir une viscosité plus élevée. L'ajout du polyamide gélifiant permet d'obtenir une application localisée et montre une bonne couvrance de la teinte. Le fini est plus mat, la teinte plus homogène que la crème de référence sans polyamide.

### 15) Stick à lèvre

Le polyamide gélifiant de l'invention peut aussi être incorporé dans des formulations de type stick. Un essai contenant 50% du polyamide gélifiant de l'exemple 4 (SNO19025) dans du Caprylic /Capric triglycéride forme un gel très solide et transparent mais trop mou pour pouvoir être présenté en stick. Le film déposé sur les lèvres est brillant, nourrissant et confortable. Le gout du raisin ainsi formulé avec le polyamide gélifiant est neutre.

En combinant ce polyamide gélifiant avec un autre polymère gélifiant d'huile, le castor oil/IPDI copolymer, on obtient un raisin de solidité acceptable et très transparent.

**Tableau 20**

| **INCI** | **Formulation avec le composé de l'exemple 4 %** | **Formulation avec le composé de l'exemple 4 et castor oil/IPDI copolymer %** |
|---|---|---|
| Caprylic / capric triglycéride | 43 | 43 |
| Dimer dilinoleyl dimer dilinoleate | 6 | 6 |
| Composé de l'exemple 4 (SNO19025) | 50 | 20 |
| acetate tocopherol | 0,5 | 0,5 |
| Parfum | 0,5 | 0,5 |
| Titanium dioxide, mica | 0,6 | 0,01 |
| Castor oil /IPDI copolymer | 0 | 30 |

La formule combinant les deux polymères maintient les nacres en suspension de manière homogène, le stick est bien solide tout en permettant une application facile du produit. Le film déposé sur les lèvres est brillant, nourrissant et confortable. Le gout du raisin ainsi formulé avec le polyamide gélifiant est neutre.

L'association du polyamide selon l'invention avec un autre polymère permet de formuler un stick totalement transparent suffisamment solide pour être présenté en stick et suffisamment plastique pour être appliqué facilement sur les lèvres.

### 16) Gelée exfoliante corporelle

**Tableau 21**

| **INCI** | **%** |
|---|---|
| Caprylic/capric triglycéride | 75,45 |
| Composé de l'exemple 4 (SNO19025) | 8,00 |
| Luffa Cylindrica Fruit | 4,00 |
| vegetable wax | 2,00 |
| Polyglyceryl-3 Oleate | 5,00 |
| Parfum | 0,50 |
| Acetate de tocopherol | 0,05 |
| C10-18 triglycérides | 5,00 |

Le polyamide de l'exemple 4 (SNO19025) permet de gélifier le mélange et de maintenir les agents exfoliants en suspension. Le gel est facile à étaler et présente une action exfoliante marquée. Il est facile à rincer et peut se présenter en tube.

### 17) Gelée solaire au Zinc

Le polyamide gélifiant de l'invention ayant un bon pouvoir suspensif, il permet de formuler des produits solaires minéraux sans eau.

**Tableau 22**

| **INCI** | **%** |
|---|---|
| Zinc Oxide (and) Caprylic/Capric Triglycéride (and) Polyhydroxystearic Acid (and) Polyglyceryl-3 Polyricinoleate (and) Isostearic Acid (and) Lecithin | 15 |
| Composé de l'exemple 4 (SNO19025) | 10 |
| Caprylic/Capric Triglycéride | 54,5 |
| Acetate tocopherol | 0,5 |
| Prunus domestica oil | 20 |

Le gel est homogène et maintient bien l'oxyde de zinc en suspension. Onctueux à l'application, il s'étale bien et dépose un film doux et poudré.

### 18) Crème solaire avec filtres organiques

Les propriétés du polyamide gélifiant selon l'invention, à savoir :
- sa haute compatibilité avec les agents protecteurs UV,
- sa capacité à stabiliser les émulsions,
- son étalement facile et homogène, et
- sa capacité à laisser un film homogène et perceptible sur la peau, en font un ingrédient intéressant dans les formules de crèmes solaires.

**Tableau 23**

| **INCI** | **%** |
|---|---|
| PEG-30 DIPOLYHYDROXYSTEARATE | 3 |
| POLYGLYCERYL-6 DIISOTEARATE | 2 |
| Composé de l'exemple 4 (SNO19025) | 4 |
| dicaprylyl carbonate | 8 |
| Homosalate | 10 |
| Coco-Caprylate/Caprate | 6 |
| Ethylhexyl Salicylate | 5 |
| Polysilicone-15 | 3 |
| Bis-Ethylhexyloxyphenol Methoxyphenyl Triazine | 4 |
| Coco-Caprylate | 8 |
| AQUA | 43 |
| GLYCERIN | 3 |
| MAGNESIUM SULFATE | 0,7 |
| TETRASODIUM EDTA | 0,05 |
| Phenoxyethanol, caprylyl glycol | 0,8 |
| Poloxamer 338, PPG12/SMDI Copolymer | 0,3 |

Cette formule s'émulsionne facilement, sa viscosité est de 4,0 Pa.s. Le polyamide gélifiant participe à augmenter la viscosité et à stabiliser l'émulsion formée. L'étalement est agréable et laisse un film protecteur très doux perceptible après application.

### 19) Crème solaire avec filtres minéraux

Une formulation de crème solaire est préparée en utilisant uniquement des filtres minéraux de grade non nanométrique (c'est-à-dire dont la taille de particule primaire n'est pas inférieure à 100nm).

**Tableaux 24**

| **INCI** | **%** |
|---|---|
| Polyglyceryl-2, Dipolyhydroxystearate | 4 |
| Shea butter | 1 |
| Caprylic/Capric Triglycéride | 25 |
| Composé de l'exemple 4 (SNO19025) | 6 |
| Titanium Dioxide | 12 |
| Zinc Oxide (and) Caprylic/Capric Triglycéride (and) Polyhydroxystearic Acid (and) Polyglyceryl-3 Polyricinoleate (and) Isostearic Acid (and) Lecithin | 23 |
| Aqua | 24 |
| Sodium chloride | 0,3 |
| Glycerine | 3 |
| Xanthan Gum | 0,2 |
| Caprylhydroxamic Acid (and) Caprylyl Glycol (and) Propanediol | 1,5 |

Le polyamide gélifiant participe à augmenter la viscosité et à stabiliser l'émulsion formée. L'émulsion hautement naturelle, se forme très facilement malgré la très forte concentration en filtres minéraux. Cette formule laisse un film couvrant protecteur lors de l'application.

### 20) Gelée démaquillante

**Tableau 25**

| **INCI** | **%** |
|---|---|
| Coryllus avellana oil | 20 |
| Squalane | 10 |
| Caprylic/Capric Triglycéride | 41,3 |
| Composé de l'exemple 4 (SNO19025) | 5 |
| Methyl Glucose Dioleate | 10 |
| Tocopheryl Acétate | 0,2 |
| Parfum | 0,5 |

Le gel formé est totalement transparent. Sa texture est onctueuse et à mémoire de forme lors de la trituration. Il s'applique bien sur le visage et retire facilement le maquillage, même waterproof. Sa capacité filmifiante permet de faciliter le démaquillage. Le résidu après démaquillage est facile à rincer à l'eau claire.

### 21) Gloss

Le polyamide gélifiant de l'invention présente un fini après étalement très brillant filmifiant à forte concentration et une onctuosité qui en font un ingrédient de choix pour les produits de maquillage type gloss.

**Tableau 26**

| **INCI** | **%** |
|---|---|
| Ricinus communis seed oil | 34 |
| Isopropyl palmitate | 25 |
| Caprylic / capric triglycéride | 30 |
| Composé de l'exemple 4 (SNO19025) | 10 |
| acétate tocopherol | 0,5 |
| Parfum | 0,5 |
| Mica, titanium dioxide | 0,05 |

Le gel formé se formule facilement. Il est parfaitement transparent et homogène, permet de maintenir les nacres en suspension, s'applique facilement sur les lèvres, il a un goût neutre et laisse un film riche et nourrissant sur les lèvres.

## Revendications

1. Composé polyamide apte à gélifier des huiles, de formule (1) 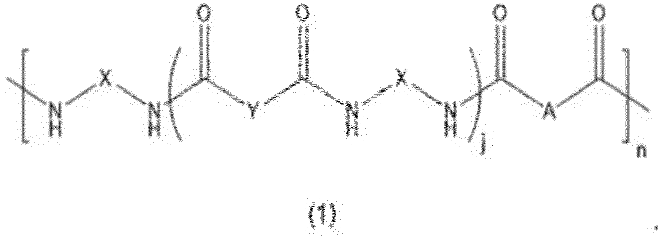 dans laquelle 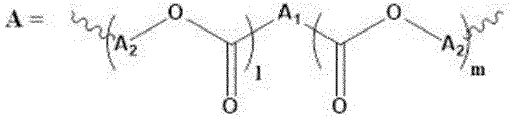
- A₁ représente un radical alkyl, linéaire ou ramifié, comprenant de 2 à 40 atomes de carbone, de préférence de 2 à 12, et en particulier de 2 à 6, ledit radical comprenant éventuellement une ou plusieurs insaturations, étant éventuellement interrompu par au moins un hétéroatome choisi parmi O, N et S, et étant éventuellement substitué par au moins un substituant -OAlk, Alk représentant un groupe alkyle comprenant de 1 à 10 atomes de carbone ;
- A₂ représente un radical alkyl, linéaire ou ramifié, comprenant de 2 à 30 atomes de carbone, de préférence de 5 à 22, ledit radical comprenant éventuellement une ou plusieurs insaturations, et étant éventuellement substitué par au moins un substituant
- OAlk, Alk représentant un groupe alkyle comprenant de 1 à 10 atomes de carbone ;
- le signe 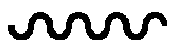 représente une liaison du segment considéré avec la chaîne principale ;
- l et m représentent, indépendamment l'un de l'autre, un nombre entier de 1 à 20, de préférence de 1 à 10, la somme l+m étant, de préférence, de 2 à 20 ;
- n est un nombre entier de 1 à 20, de préférence de 2 à 8 ;
- j est un nombre entier de 1 à 10, de préférence de 1 à 3 ;
- X représente un radical alkyl, linéaire ou ramifié, comprenant de 2 à 60 atomes de carbone, de préférence 16 à 45 atomes de carbone, ledit radical comprenant éventuellement une ou plusieurs insaturations ;
- Y représente un radical alkyl, linéaire ou ramifié, comprenant de 3 à 35 atomes de carbone, de préférence 6 à 18 atomes de carbone, ledit radical comprenant éventuellement une ou plusieurs insaturations, ou un hétérocycle ayant 3 à 10 membres et comprenant 1 ou 2 hétéroatome(s) choisi(s) parmi O, N et S.

2. Composé polyamide de formule (1) selon la revendication 1 dans lequel A₁ représente un radical alkyl linéaire comprenant de 2 à 40, de préférence de 2 à 12, en particulier 3 atomes de carbone, ledit radical comprenant éventuellement une ou plusieurs insaturations.

3. Composé polyamide de formule (1) selon la revendication 1 ou 2, dans lequel A₂ représente un radical alkyl, linéaire ou ramifié, comprenant de 5 à 18 atomes de carbone, ledit radical comprenant éventuellement une ou plusieurs insaturations, et, de préférence, un groupe de formule

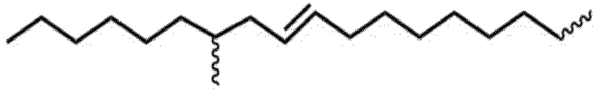

4. Composé polyamide de formule (I) selon l'une des revendications 1 à 3, dans lequel la somme l+m a une valeur moyenne de 7, 11 ou 15.

5. Composé polyamide de formule (1) selon l'une des revendications 1 à 3, dans laquelle :
- A₁ représente un radical alkyl linéaire comprenant 3 atomes de carbone;
- A₂ représente un radical alkyl ramifié comprenant 17 atomes de carbone;
- l et m représentent, indépendamment l'un de l'autre, un nombre entier de 3 à 4, la somme l+m étant, de préférence, de 6 à 8;
- n est un nombre entier de 1 à 8, de préférence de 1 à 3;
- j est un nombre entier de 1 à 10, de préférence de 1 à 3 ;
- X représente un radical alkyl ramifié comprenant 36 atomes de carbone;
- Y représente un radical alkyl linéaire comprenant 8 atomes de carbone.

6. Procédé de préparation d'un composé polyamide de formule (1) 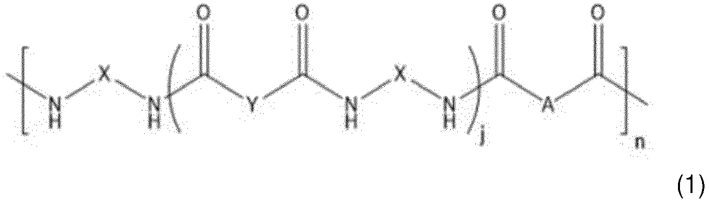 dans laquelle 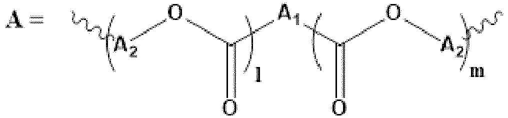
- A₁ représente un radical alkyl, linéaire ou ramifié, comprenant de 2 à 40 atomes de carbone, de préférence de 2 à 12, et en particulier de 2 à 6, ledit radical comprenant éventuellement une ou plusieurs insaturations, étant éventuellement interrompu par au moins un hétéroatome choisi parmi O, N et S, et étant éventuellement substitué par au moins un substituant -OAlk, Alk représentant un groupe alkyle comprenant de 1 à 10 atomes de carbone ;
- A₂ représente un radical alkyl, linéaire ou ramifié, comprenant de 2 à 30 atomes de carbone, de préférence de 5 à 22, ledit radical comprenant éventuellement une ou plusieurs insaturations, et étant éventuellement substitué par au moins un substituant
- OAlk, Alk représentant un groupe alkyle comprenant de 1 à 10 atomes de carbone ;
- le signe 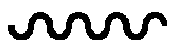 représente une liaison du segment considéré avec la chaîne principale
- l et m représentent, indépendamment l'un de l'autre, un nombre entier de 1 à 20, de préférence de 1 à 10, la somme l+m étant, de préférence, de 2 à 20 ;
- n est un nombre entier de 1 à 20, de préférence de 2 à 8 ;
- j est un nombre entier de 1 à 10, de préférence de 1 à 3 ;
- X représente un radical alkyl, linéaire ou ramifié, comprenant de 2 à 60 atomes de carbone, de préférence 16 à 45 atomes de carbone, ledit radical comprenant éventuellement une ou plusieurs insaturations ;
- Y représente un radical alkyl, linéaire ou ramifié, comprenant de 3 à 35 atomes de carbone, de préférence 6 à 18 atomes de carbone, ledit radical comprenant éventuellement une ou plusieurs insaturations, ou un hétérocycle ayant 3 à 10 membres et comprenant 1 ou 2 hétéroatome(s) choisi(s) parmi O, N et S, comprenant les étapes suivantes :
1) La préparation d'un précurseur polyamide par fonctionnalisation d'un diacide de formule (2) par une diamine de formule (3), pour obtenir un polyamide diamine de formule (4) 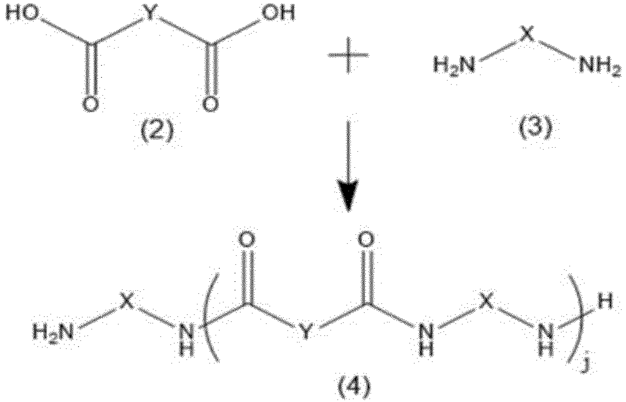 dans lesquelles j, X et Y sont tels que définis ci-dessus;
2) La modification d'un polyester diacide de formule (5) 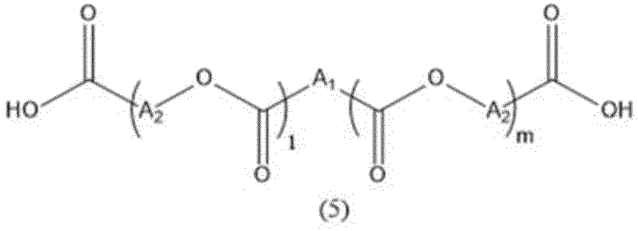 par ajout du polyamide diamine de formule (4) 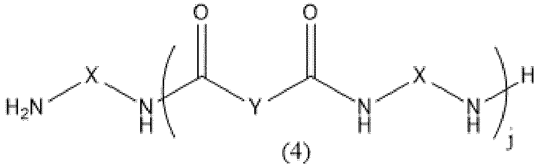 dans lesquelles A₁, A₂, X, Y, I, m, n et j sont tels que définis ci-dessus, conduisant au polyamide de formule (1) ci-dessus.

7. Procédé selon la revendication 6, dans lequel la 1^{ère} étape est effectuée par mélange d'un équivalent de diacide de formule (2) et d'une quantité de diamine de formule (3) comprise entre 1 et 4 équivalents, à une température comprise entre 100°C et 240°C, de préférence entre 130°C et 150°C.

8. Procédé selon l'une des revendications 6 ou 7, dans lequel l'étape de modification de polyester diacide de formule (5) par ajout polyamide diamine de formule (4) conduisant à un polyamide gélifiant de formule (1) est effectuée en ajoutant 1 équivalent de polyester diacide sur une quantité de précurseur gélifiant (4) comprise entre 0,2 et 1 équivalent, à une température comprise entre 100 et 240°C, de préférence entre 130 et 150°C.

9. Procédé selon l'une quelconque des revendications 6 à 8, comprend en outre, une étape additionnelle comprenant la terminaison des chaînes du composé polyamide de formule (1) par ajout d'un composé de terminaison alcool de formule (6), conduisant à un composé polyamide de formule (7): 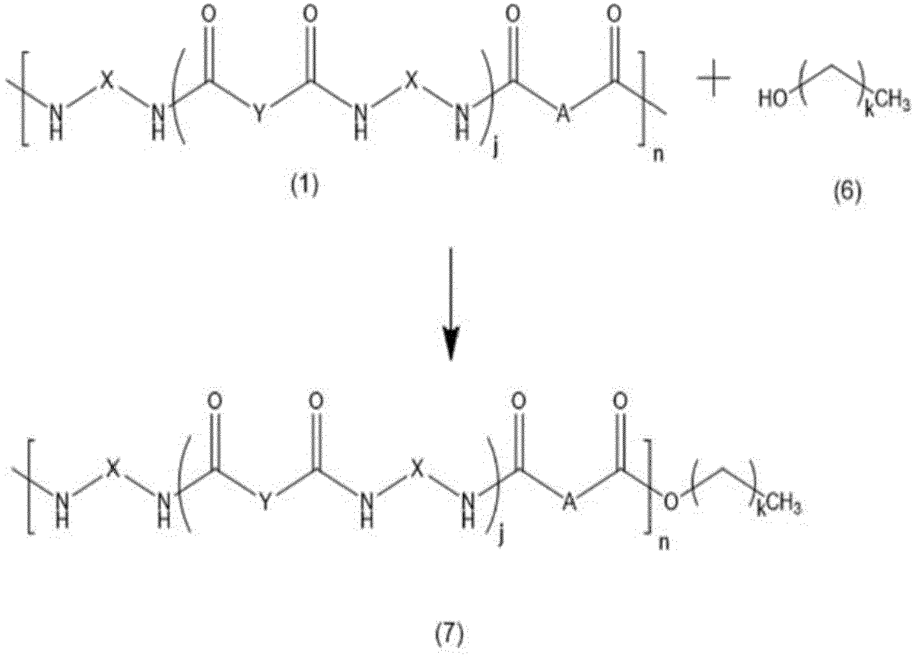 dans lesquelles : 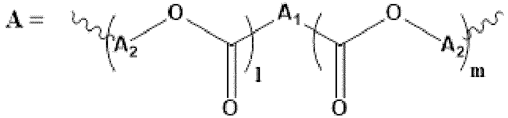
- A, X, Y, A, A1, A2, j, n sont tels que définis dans la revendication 6 ;
- k est un nombre entier de 2 à 60, de préférence de 8 à 40.

10. Composé polyamide de formule (7) 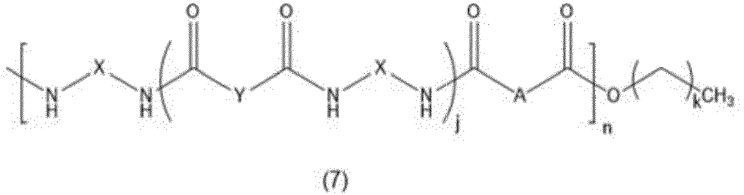 dans laquelle : 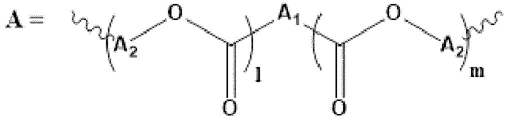
- A₁ représente un radical alkyl, linéaire ou ramifié, comprenant de 2 à 40 atomes de carbone, de préférence de 2 à 12, et en particulier de 2 à 6, ledit radical comprenant éventuellement une ou plusieurs insaturations, étant éventuellement interrompu par au moins un hétéroatome choisi parmi O, N et S, et étant éventuellement substitué par au moins un substituant -OAlk, Alk représentant un groupe alkyle comprenant de 1 à 10 atomes de carbone ;
- A₂ représente un radical alkyl, linéaire ou ramifié, comprenant de 2 à 30 atomes de carbone, de préférence de 5 à 22, ledit radical comprenant éventuellement une ou plusieurs insaturations, et étant éventuellement substitué par au moins un substituant
- OAlk, Alk représentant un groupe alkyle comprenant de 1 à 10 atomes de carbone ;
- le signe 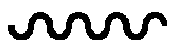 représente une liaison du segment considéré avec la chaîne principale
- l et m représentent, indépendamment l'un de l'autre, un nombre entier de 1 à 20, de préférence de 1 à 10, la somme l+m étant, de préférence, de 2 à 20 ;
- n est un nombre entier de 1 à 20, de préférence de 1 à 8 ;
- j est un nombre entier de 1 à 10, de préférence de 1 à 3 ;
- k est un nombre entier de 2 à 60, de préférence de 8 à 40 ;
- X représente un radical alkyl, linéaire ou ramifié, comprenant de 2 à 60 atomes de carbone, de préférence 16 à 45 atomes de carbone, ledit radical comprenant éventuellement une ou plusieurs insaturations ;
- Y représente un radical alkyl, linéaire ou ramifié, comprenant de 3 à 35 atomes de carbone, de préférence 6 à 18 atomes de carbone, ledit radical comprenant éventuellement une ou plusieurs insaturations, ou un hétérocycle ayant 3 à 10 membres et comprenant 1 ou 2 hétéroatome(s) choisi(s) parmi O, N et S.

11. Utilisation du polyamide gélifiant de formule (1) ou (7) selon l'une quelconque des revendications 1 à 5 ou 10, ou obtenu selon le procédé de l'une quelconque des revendications 6 à 9, comme agent gélifiant d'huiles.

12. Gels contenant ou formés à partir du polyamide gélifiant selon polyamide gélifiant de formule (1) ou (7) selon l'une quelconque des revendications 1 à 5 ou 10, ou obtenu selon le procédé de l'une quelconque des revendications 6 à 9.

13. Gels selon la revendication 12, dans lesquels la teneur massique du polyamide gélifiant est comprise entre 0,5% et 10%, de préférence de 3 à 8%.

14. Composition pharmaceutique ou cosmétique comprenant un polyamide gélifiant de formule (1) ou (7) selon l'une quelconque des revendications 1 à 5 ou 10, ou obtenu selon le procédé de l'une quelconque des revendications 6 à 9, ou les gels formés à partir desdits polyamides gélifiants selon l'une des revendications 12 ou 13.

15. Composition cosmétique comprenant les gels formés à partir du polyamide gélifiant de formule (1) ou (7) selon l'une quelconque des revendications 1 à 5 ou 10, ou obtenu selon le procédé de l'une quelconque des revendications 6 à 9, dans laquelle la teneur massique du polyamide gélifiant est comprise entre 0,5% et 10%, de préférence de 3 à 8%

## Patentansprüche

1. Polyamidverbindung, die Öle gelieren kann, der Formel (1): 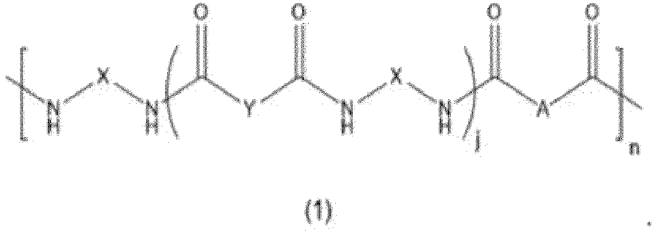 in der 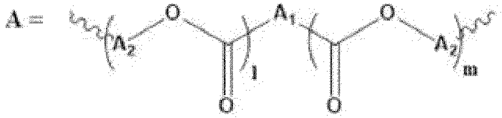
- A₁ für einen Alkylrest, gerade oder verzweigt, steht, der von 2 bis 40 Kohlenstoffatome, vorzugsweise von 2 bis 12 und insbesondere von 2 bis 6 umfasst, wobei der Rest gegebenenfalls eine oder mehrere Ungesättigtheiten umfasst, gegebenenfalls durch mindestens ein Heteroatom unterbrochen ist, das aus O, N und S ausgewählt ist, und gegebenenfalls durch mindestens einen Substituenten -Oalk substituiert ist, wobei Alk eine Alkylgruppe darstellt, die von 1 bis 10 Kohlenstoffatome umfasst;
- A₂ für einen Alkylrest, gerade oder verzweigt, steht, der von 2 bis 30 Kohlenstoffatome, vorzugsweise von 5 bis 22 umfasst, wobei der Rest gegebenenfalls eine oder mehrere Ungesättigtheiten umfasst und gegebenenfalls durch mindestens einen Substituenten -Oalk substituiert ist, wobei Alk eine Alkylgruppe darstellt, die von 1 bis 10 Kohlenstoffatome umfasst;
- das Symbol 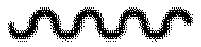 für eine Bindung des betrachteten Segments mit der Hauptkette steht;
- 1 und m unabhängig voneinander für eine ganze Zahl von 1 bis 20, vorzugsweise von 1 bis 10 stehen, wobei die Summe 1 + m vorzugsweise von 2 bis 20 beträgt;
- n eine ganze Zahl von 1 bis 20, vorzugsweise von 2 bis 8 ist;
- j eine ganze Zahl von 1 bis 10, vorzugsweise von 1 bis 3 ist;
- X für einen Alkylrest, gerade oder verzweigt, steht, der von 2 bis 60 Kohlenstoffatome, vorzugsweise von 16 bis 45 Kohlenstoffatome umfasst, wobei der Rest gegebenenfalls eine oder mehrere Ungesättigtheiten umfasst;
- Y für einen Alkylrest, gerade oder verzweigt, der von 3 bis 35 Kohlenstoffatome, vorzugsweise von 6 bis 18 Kohlenstoffatome umfasst, wobei der Rest gegebenenfalls eine oder mehrere Ungesättigtheiten umfasst, oder einen Heterocyclus, der 3 bis 10 Mitglieder aufweist und 1 oder 2 Heteroatome umfasst, die aus O, N und S ausgewählt sind, steht.

2. Polyamidverbindung der Formel (1) nach Anspruch 1, wobei A₁ für einen geraden Alkylrest steht, der von 2 bis 40, vorzugsweise 2 bis 12, insbesondere 3 Kohlenstoffatome umfasst, wobei der Rest gegebenenfalls eine oder mehrere Ungesättigtheiten umfasst.

3. Polyamidverbindung der Formel (1) nach Anspruch 1 oder 2, wobei A₂ für einen Alkylrest, gerade oder verzweigt, steht, der von 5 bis 18 Kohlenstoffatome umfasst, wobei der Rest gegebenenfalls eine oder mehrere Ungesättigtheiten und vorzugsweise eine Gruppe der Formel: 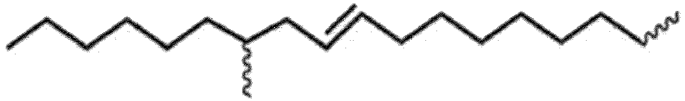 umfasst.

4. Polyamidverbindung der Formel (1) nach einem der Ansprüche 1 bis 3, wobei die Summe 1 + m einen Mittelwert von 7, 11 oder 15 aufweist.

5. Polyamidverbindung der Formel (1) nach einem der Ansprüche 1 bis 3, in der:
- A₁ für einen geraden Alkylrest steht, der 3 Kohlenstoffatome umfasst;
- A₂ für einen verzweigten Alkylrest steht, der 17 Kohlenstoffatome umfasst;
- 1 und m unabhängig voneinander für eine ganze Zahl von 3 bis 4 stehen, wobei die Summe 1 + m vorzugsweise von 6 bis 8 beträgt;
- n eine ganze Zahl von 1 bis 8, vorzugsweise von 1 bis 3 ist;
- j eine ganze Zahl von 1 bis 10, vorzugsweise von 1 bis 3 ist;
- X für einen verzweigten Alkylrest steht, der 36 Kohlenstoffatome umfasst;
- Y für einen geraden Alkylrest steht, der 8 Kohlenstoffatome umfasst.

6. Verfahren zur Herstellung einer Polyamidverbindung der Formel (1): 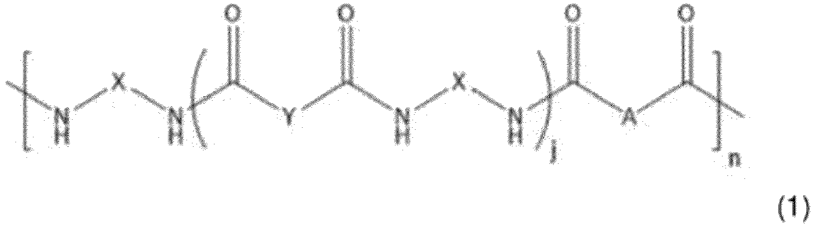 in der 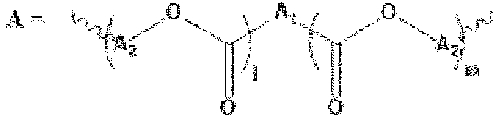
- A₁ für einen Alkylrest, gerade oder verzweigt, steht, der von 2 bis 40 Kohlenstoffatome, vorzugsweise von 2 bis 12 und insbesondere von 2 bis 6 umfasst, wobei der Rest gegebenenfalls eine oder mehrere Ungesättigtheiten umfasst, gegebenenfalls durch mindestens ein Heteroatom unterbrochen ist, das aus O, N und S ausgewählt ist, und gegebenenfalls durch mindestens einen Substituenten -Oalk substituiert ist, wobei Alk eine Alkylgruppe darstellt, die von 1 bis 10 Kohlenstoffatome umfasst;
- A₂ für einen Alkylrest, gerade oder verzweigt, steht, der von 2 bis 30 Kohlenstoffatome, vorzugsweise von 5 bis 22 umfasst, wobei der Rest gegebenenfalls eine oder mehrere Ungesättigtheiten umfasst und gegebenenfalls durch mindestens einen Substituenten -Oalk substituiert ist, wobei Alk eine Alkylgruppe darstellt, die von 1 bis 10 Kohlenstoffatome umfasst;
- das Symbol 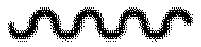 für eine Bindung des betrachteten Segments mit der Hauptkette steht;
- 1 und m unabhängig voneinander für eine ganze Zahl von 1 bis 20, vorzugsweise von 1 bis 10 stehen, wobei die Summe 1 + m vorzugsweise von 2 bis 20 beträgt;
- n eine ganze Zahl von 1 bis 20, vorzugsweise von 2 bis 8 ist;
- j eine ganze Zahl von 1 bis 10, vorzugsweise von 1 bis 3 ist;
- X für einen Alkylrest, gerade oder verzweigt, steht, der von 2 bis 60 Kohlenstoffatome, vorzugsweise von 16 bis 45 Kohlenstoffatome umfasst, wobei der Rest gegebenenfalls eine oder mehrere Ungesättigtheiten umfasst;
- Y für einen Alkylrest, gerade oder verzweigt, der von 3 bis 35 Kohlenstoffatome, vorzugsweise von 6 bis 18 Kohlenstoffatome umfasst, wobei der Rest gegebenenfalls eine oder mehrere Ungesättigtheiten umfasst, oder einen Heterocyclus, der 3 bis 10 Mitglieder aufweist und 1 oder 2 Heteroatome umfasst, die aus O, N und S ausgewählt sind, steht,
umfassend die folgenden Schritte:
1) die Herstellung eines Polyamidvorläufers durch Funktionalisierung einer Disäure der Formel (2) mit einem Diamin der Formel (3), um ein Diaminpolyamid der Formel (4) zu erhalten: 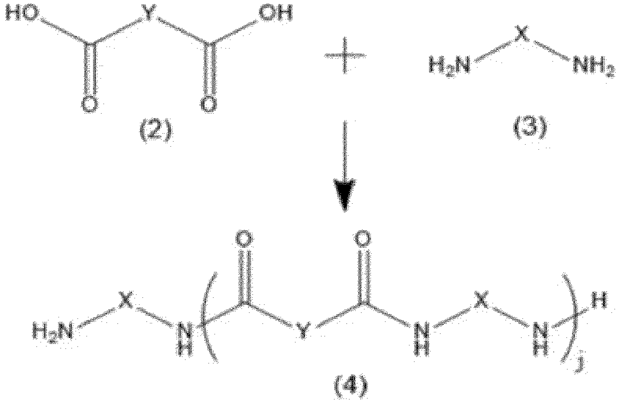 in denen j, X und Y wie oben definiert sind;
2) die Modifikation eines Disäurepolyesters der Formel (5) : 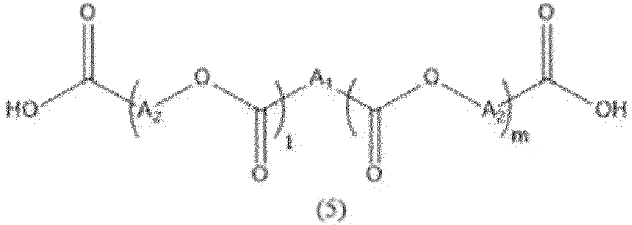 durch Zugabe des Diaminpolyamids der Formel (4): 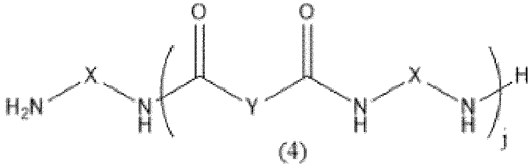 in denen A₁, A₂, X, Y, 1, m, n und j wie oben definiert sind,
was zu dem obigen Polyamid der Formel (1) führt.

7. Verfahren nach Anspruch 6, in dem der 1. Schritt durch Mischung eines Äquivalents der Disäure der Formel (2) und einer Menge des Diamins der Formel (3), die zwischen 1 und 4 Äquivalenten umfasst, bei einer Temperatur, die zwischen 100 °C und 240 °C, vorzugsweise zwischen 130 °C und 150 °C liegt, durchgeführt wird.

8. Verfahren nach einem der Ansprüche 6 oder 7, in dem der Schritt der Modifikation des Disäurepolyesters der Formel (5) durch Zugabe des Diaminpolyamids der Formel (4), der zu einem gelierenden Polyamid der Formel (1) führt, durch Zugaben von 1 Äquivalent des Disäurepolyesters zu einer Menge des gelierenden Vorläufers (4), die zwischen 0,2 und 1 Äquivalent umfasst, bei einer Temperatur, die zwischen 100 °C und 240 °C, vorzugsweise zwischen 130 °C und 150 °C liegt, durchgeführt wird.

9. Verfahren nach einem der Ansprüche 6 bis 8, das weiterhin einen zusätzlichen Schritt umfasst, der den Kettenabbruch der Polyamidverbindung der Formel (1) durch Zugabe einer Alkohol-Abbruchverbindung der Formel (6) umfasst, was zu einer Polyamidverbindung der Formel (7) führt: 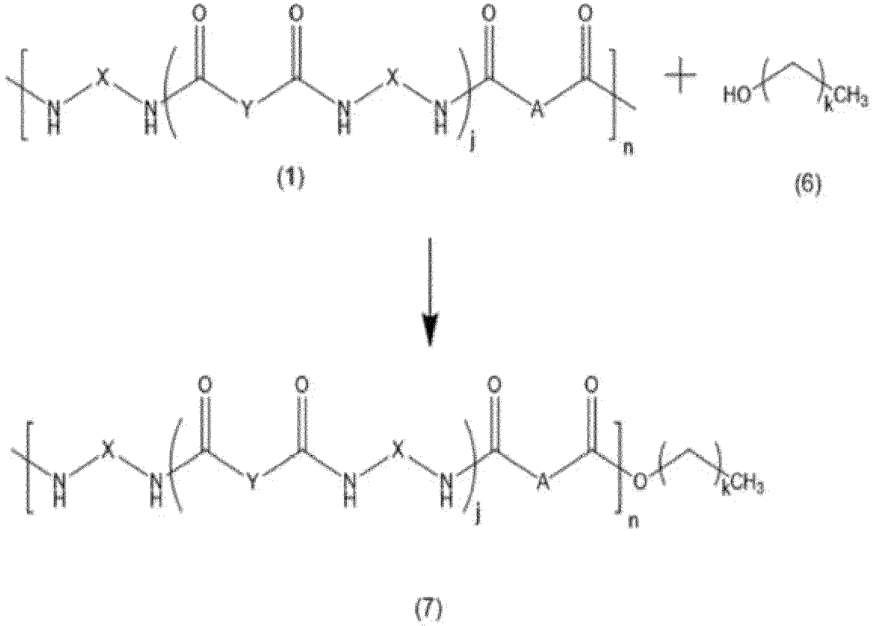 in denen: 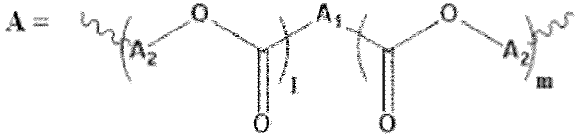
- A, X, Y, A, A1, A2, j, n wie im Anspruch 6 definiert sind;
- k eine ganze Zahl von 2 bis 60, vorzugsweise von 8 bis 40 ist.

10. Polyamidverbindung der Formel (7): 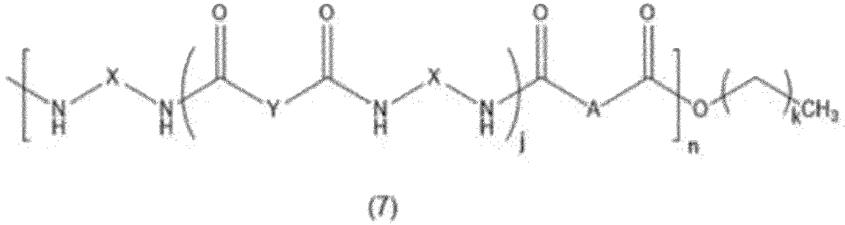 in der: 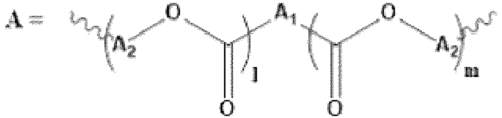
- A₁ für einen Alkylrest, gerade oder verzweigt, steht, der von 2 bis 40 Kohlenstoffatome, vorzugsweise von 2 bis 12 und insbesondere von 2 bis 6 umfasst, wobei der Rest gegebenenfalls eine oder mehrere Ungesättigtheiten umfasst, gegebenenfalls durch mindestens ein Heteroatom unterbrochen ist, das aus O, N und S ausgewählt ist, und gegebenenfalls durch mindestens einen Substituenten -OAlk substituiert ist, wobei Alk eine Alkylgruppe darstellt, die von 1 bis 10 Kohlenstoffatome umfasst;
- A₂ für einen Alkylrest, gerade oder verzweigt, steht, der von 2 bis 30 Kohlenstoffatome, vorzugsweise von 5 bis 22 umfasst, wobei der Rest gegebenenfalls eine oder mehrere Ungesättigtheiten umfasst und gegebenenfalls durch mindestens einen Substituenten -OAlk substituiert ist, wobei Alk eine Alkylgruppe darstellt, die von 1 bis 10 Kohlenstoffatome umfasst;
- das Symbol 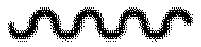 für eine Bindung des betrachteten Segments mit der Hauptkette steht;
- 1 und m unabhängig voneinander für eine ganze Zahl von 1 bis 20, vorzugsweise von 1 bis 10 stehen, wobei die Summe 1 + m vorzugsweise von 2 bis 20 beträgt;
- n eine ganze Zahl von 1 bis 20, vorzugsweise von 1 bis 8 ist;
- j eine ganze Zahl von 1 bis 10, vorzugsweise von 1 bis 3 ist;
- k eine ganze Zahl von 2 bis 60, vorzugsweise von 8 bis 40 ist;
- X für einen Alkylrest, gerade oder verzweigt, steht, der von 2 bis 60 Kohlenstoffatome, vorzugsweise von 16 bis 45 Kohlenstoffatome umfasst, wobei der Rest gegebenenfalls eine oder mehrere Ungesättigtheiten umfasst;
- Y für einen Alkylrest, gerade oder verzweigt, der von 3 bis 35 Kohlenstoffatome, vorzugsweise von 6 bis 18 Kohlenstoffatome umfasst, wobei der Rest gegebenenfalls eine oder mehrere Ungesättigtheiten umfasst, oder einen Heterocyclus, der 3 bis 10 Mitglieder aufweist und 1 oder 2 Heteroatome umfasst, die aus O, N und S ausgewählt sind, steht.

11. Verwendung eines gelierenden Polyamids der Formel (1) oder (7) nach einem der Ansprüche 1 bis 5 oder 10 oder eines gemäß dem Verfahren nach einem der Ansprüche 6 bis 9 erhaltenen als Ölgeliermittel.

12. Gele, die das gelierende Polyamid gemäß einem gelierenden Polyamid der Formel (1) oder (7) nach einem der Ansprüche 1 bis 5 oder 10 oder einem gemäß dem Verfahren nach einem der Ansprüche 6 bis 9 erhaltenen enthalten oder zum Teil davon gebildet werden.

13. Gele nach Anspruch 12, in denen der Gewichtsgehalt des gelierenden Polyamids zwischen 0,5 % und 10 % liegt, vorzugsweise von 3 bis 8 % beträgt.

14. Pharmazeutische oder kosmetische Zusammensetzung, die ein gelierendes Polyamid der Formel (1) oder (7) nach einem der Ansprüche 1 bis 5 oder 10 oder ein gemäß dem Verfahren nach einem der Ansprüche 6 bis 9 erhaltenes oder die Gele, die zum Teil von den gelierenden Polyamiden gebildet werden, nach einem der Ansprüche 12 oder 13 umfasst.

15. Kosmetische Zusammensetzung, die die Gele, die zum Teil von dem gelierenden Polyamid der Formel (1) oder (7) nach einem der Ansprüche 1 bis 5 oder 10 oder dem gemäß dem Verfahren nach einem der Ansprüche 6 bis 9 erhaltenen gebildet werden, umfasst, in der der Gewichtsgehalt des gelierenden Polyamids zwischen 0,5 % und 10 % liegt, vorzugsweise von 3 bis 8 % beträgt.

## Claims

1. Polyamide compound able to gel oils, of formula (1) 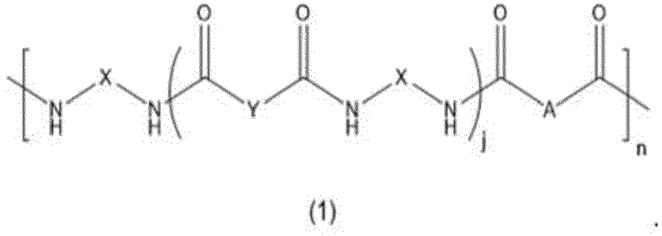 wherein 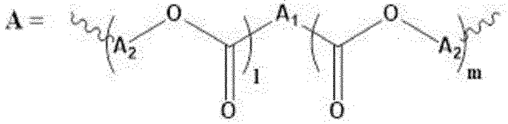
- A₁ represents an alkyl radical, linear or branched, comprising from 2 to 40 carbon atoms, preferably from 2 to 12, and in particular from 2 to 6, said radical optionally comprising one or more unsaturations, being optionally interrupted by at least one heteroatom selected from O, N and S, and being optionally substituted by at least one
- OAlk substituent, Alk representing an alkyl group comprising from 1 to 10 carbon atoms;
- A₂ represents an alkyl radical, linear or branched, comprising from 2 to 30 carbon atoms, preferably from 5 to 22, said radical optionally comprising one or more unsaturations, and being optionally substituted by at least one -OAlk substituent, Alk representing an alkyl group comprising from 1 to 10 carbon atoms;
- the sign 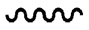 represents a bond of the segment in question with the main chain;
- I and m represent, independently of each other, an integer from 1 to 20, preferably from 1 to 10, the sum I + m preferably being from 2 to 20;
- n is an integer from 1 to 20, preferably from 2 to 8;
- j is an integer from 1 to 10, preferably from 1 to 3;
- X represents an alkyl radical, linear or branched, comprising from 2 to 60 carbon atoms, preferably 16 to 45 carbon atoms, said radical optionally comprising one or more unsaturations;
- Y represents an alkyl radical, linear or branched, comprising from 3 to 35 carbon atoms, preferably from 6 to 18 carbon atoms, said radical optionally comprising one or more unsaturations, or a heterocycle having 3 to 10 members and comprising 1 or 2 heteroatoms selected from O, N and S.

2. Polyamide compound of formula (1) according to claim 1, wherein A₁ represents a linear alkyl radical comprising from 2 to 40, preferably from 2 to 12, in particular 3 carbon atoms, said radical optionally comprising one or more unsaturations.

3. Polyamide compound of formula (1) according to claim 1 or 2, wherein A₂ represents an alkyl radical, linear or branched, comprising from 5 to 18 carbon atoms, said radical optionally comprising one or more unsaturations, and preferably a group of formula

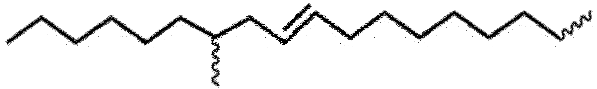

4. Polyamide compound of formula (1) according to one of claims 1 to 3, wherein the sum l+m has a mean value of 7, 11 or 15.

5. Polyamide compound of formula (1) according to one of claims 1 to 3, wherein:
- A₁ represents a linear alkyl radical comprising 3 carbon atoms;
- A₂ represents a branched alkyl radical comprising 17 carbon atoms;
- I and m represent, independently of each other, an integer from 3 to 4, the sum I + m preferably being from 6 to 8;
- n is an integer from 1 to 8, preferably from 1 to 3;
- j is an integer from 1 to 10, preferably from 1 to 3;
- X represents a branched alkyl radical comprising 36 carbon atoms;
- Y represents a linear alkyl radical comprising 8 carbon atoms.

6. Method for preparing a polyamide compound of formula (1) 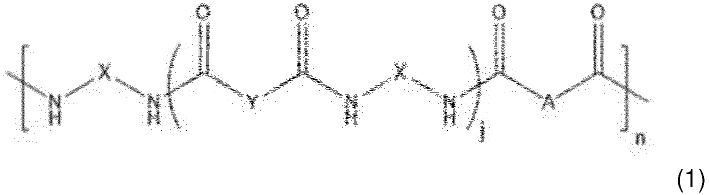 wherein 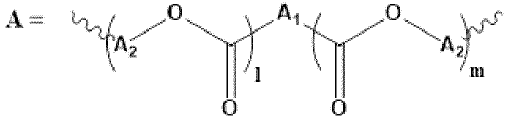
- A₁ represents an alkyl radical, linear or branched, comprising from 2 to 40 carbon atoms, preferably from 2 to 12, and in particular from 2 to 6, said radical optionally comprising one or more unsaturations, being optionally interrupted by at least one heteroatom selected from O, N and S, and being optionally substituted by at least one
- OAlk substituent, Alk representing an alkyl group comprising from 1 to 10 carbon atoms;
- A₂ represents an alkyl radical, linear or branched, comprising from 2 to 30 carbon atoms, preferably from 5 to 22, said radical optionally comprising one or more unsaturations, and being optionally substituted by at least one -OAlk substituent, Alk representing an alkyl group comprising from 1 to 10 carbon atoms;
- the sign 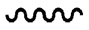 represents a bond of the segment in question with the main chain;
- I and m represent, independently of each other, an integer from 1 to 20, preferably from 1 to 10, the sum I + m preferably being from 2 to 20;
- n is an integer from 1 to 20, preferably from 2 to 8;
- j is an integer from 1 to 10, preferably from 1 to 3;
- X represents an alkyl radical, linear or branched, comprising from 2 to 60 carbon atoms, preferably 16 to 45 carbon atoms, said radical optionally comprising one or more unsaturations;
- Y represents an alkyl radical, linear or branched, comprising from 3 to 35 carbon atoms, preferably from 6 to 18 carbon atoms, said radical optionally comprising one or more unsaturations, or a heterocycle having 3 to 10 members and comprising 1 or 2 heteroatoms selected from O, N and S,
comprising the following steps:
1) Preparing a polyamide precursor by functionalizing a diacid of formula (2) by a diamine of formula (3) to obtain a diamine polyamide of formula (4): 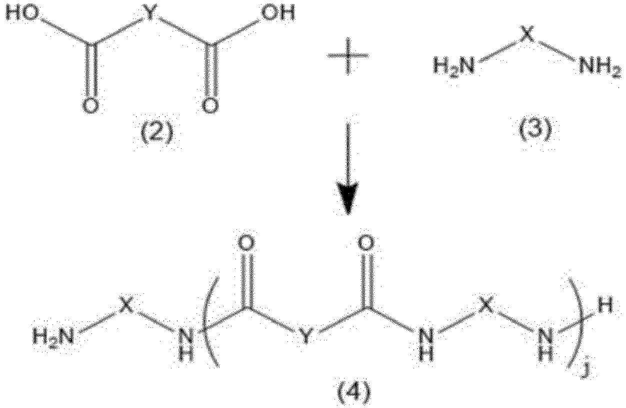 wherein j, X et Y are as defined above;
2) Modifying a diacid polyester of formula (5) 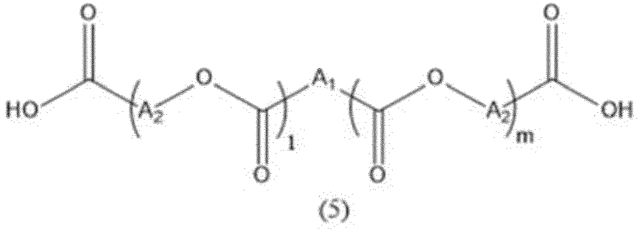 by adding the diamine polyamide of formula (4) 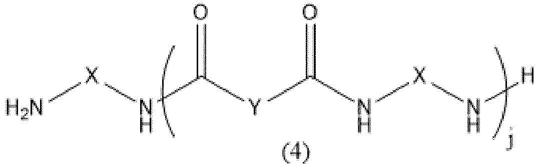 wherein A₁, A₂, X, Y, l, m, n and j are as defined above,
leading to the polyamide of formula (1) above.

7. Method according to claim 6, wherein the 1^{st} step is implemented by mixing an equivalent of diacid of formula (2) and a quantity of diamine of formula (3) of between 1 and 4 equivalents, at a temperature of between 100°C and 240°C, preferably between 130°C and 150°C.

8. Method according to one of claims 6 or 7, wherein the step of modifying the diacid polyester of formula (5) by adding diamine polyamide of formula (4) leading to a gelling polyamide of formula (1) is implemented by adding 1 equivalent of diacid polyester to a quantity of gelling precursor (4) of between 0.2 and 1 equivalent, at a temperature of between 100 and 240°C, preferably between 130 and 150°C.

9. Method according to any one of claims 6 to 8, furthermore comprises an additional step comprising terminating the chains of the polyamide compound of formula (1) by adding an alcohol termination compound of formula (6), leading to a polyamide compound of formula (7): 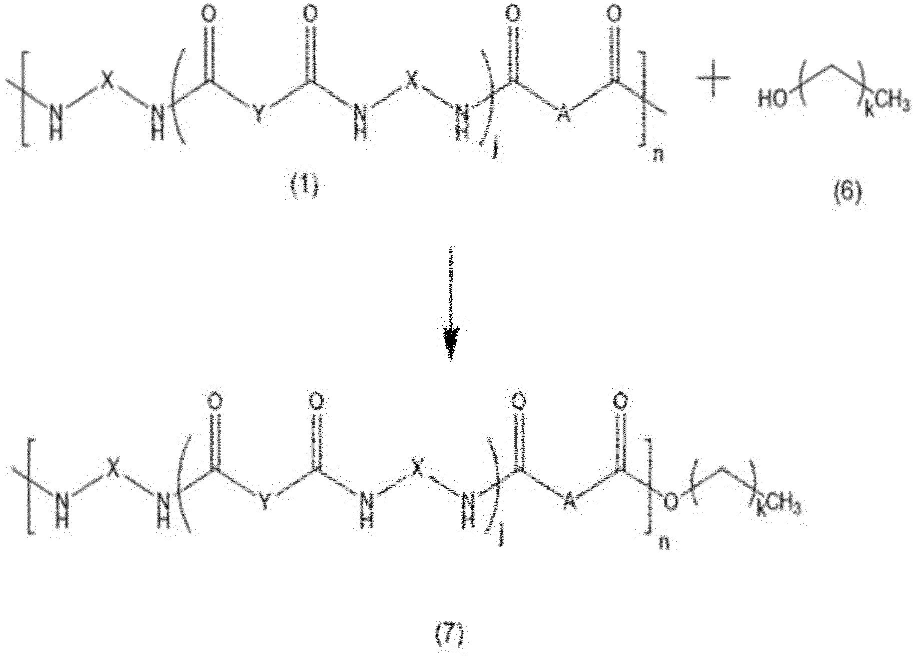 wherein: 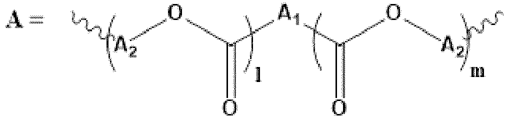
- A, X, Y, A, A1, A2, j, n are as defined in claim 6;
- k is an integer from 2 to 60, preferably from 8 to 40.

10. Polyamide compound of formula (7) 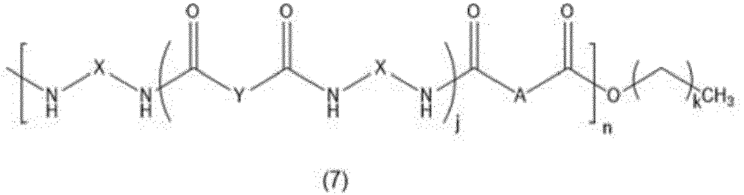 wherein: 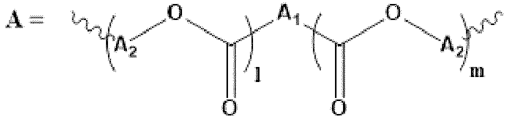
- A₁ represents an alkyl radical, linear or branched, comprising from 2 to 40 carbon atoms, preferably from 2 to 12, and in particular from 2 to 6, said radical optionally comprising one or more unsaturations, being optionally interrupted by at least one heteroatom selected from O, N and S, and being optionally substituted by at least one
- OAlk substituent, Alk representing an alkyl group comprising from 1 to 10 carbon atoms;
- A₂ represents an alkyl radical, linear or branched, comprising from 2 to 30 carbon atoms, preferably from 5 to 22, said radical optionally comprising one or more unsaturations, and being optionally substituted by at least one -OAlk substituent, Alk representing an alkyl group comprising from 1 to 10 carbon atoms;
- the sign 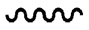 represents a bond of the segment in question with the main chain;
- I and m represent, independently of each other, an integer from 1 to 20, preferably from 1 to 10, the sum I + m preferably being from 2 to 20;
- n is an integer from 1 to 20, preferably from 1 to 8;
- j is an integer from 1 to 10, preferably from 1 to 3;
- k is an integer from 2 to 60, preferably from 8 to 40;
- X represents an alkyl radical, linear or branched, comprising from 2 to 60 carbon atoms, preferably 16 to 45 carbon atoms, said radical optionally comprising one or more unsaturations;
- Y represents an alkyl radical, linear or branched, comprising from 3 to 35 carbon atoms, preferably from 6 to 18 carbon atoms, said radical optionally comprising one or more unsaturations, or a heterocycle having 3 to 10 members and comprising 1 or 2 heteroatoms selected from O, N and S.

11. Use of the gelling polyamide of formula (1) or (7) according to any one of claims 1 to 5 or 10, or obtained according to the method of any one of claims 6 to 9, as oil-gelling agent.

12. Gels containing or formed from the gelling polyamide of formula (1) or (7) according to any one of claims 1 to 5 or 10, or obtained according to the method of any one of claims 6 to 9.

13. Gels according to claim 12, wherein the content by weight of the gelling polyamide is between 0.5% and 10%, preferably from 3 to 8%.

14. Pharmaceutical or cosmetic composition comprising a gelling polyamide of formula (1) or (7) according to any one of claims 1 to 5 or 10, or obtained according to the method of any one of claims 6 to 9, or the gels formed from said gelling polyamides according to one of claims 12 or 13.

15. Cosmetic composition comprising the gels formed from the gelling polyamide of formula (1) or (7) according to any one of claims 1 to 5 or 10, or obtained according to the method of any one of claims 6 to 9, wherein the content by weight of the gelling polyamide is between 0.5% and 10%, preferably from 3 to 8%.
